# EUROPEAN PATENT SPECIFICATION

(11) **EP 1 228 233 B1**
(45) Date of publication and mention of the grant of the patent: **10.01.2007**
(21) Application number: 00977143.7
(22) Date of filing: 10.11.2000
(51) Int. Cl.: C12N 15/86, C12N 15/62, C07K 14/435, C12N 5/10

(54) **METHODS AND COMPOSITIONS COMPRISING RENILLA GFP**
VERFAHREN AND ZUSAMMENSTELLUNGEN, DIE RENILLA GFP ENTHALTEN
PROCEDES ET COMPOSITIONS CONTENANT DES PROTEINES FLUORESCENTES VERTES (GFP) DE RENILLA

(30) Priority: 10.11.1999 US 164592 P
(43) Date of publication of application: 07.08.2002
(73) Proprietor: Rigel Pharmaceuticals, Inc., South San Francisco, CA 94080 (US)
(72) Inventor: ANDERSON, David, San Bruno, CA 94066 (US); PEELLE, Beau Robert, Locust Valley, NY 11560 (US)
(74) Representative: Kiddle, Simon John
(86) International application number: PCT/US2000/030915
(87) International publication number: WO 2001/034824

(56) References cited:
- WO-A-01/34806
- WO-A-01/68824
- WO-A-95/07463
- WO-A-97/27212
- WO-A-97/27213
- WO-A-99/49019
- ARAN JM, GOTTESMAN MM, PASTAN I.: "Construction and characterization of bicistronic retroviral vectors encoding the multidrug transporter and beta-galactosidase or green fluorescent protein." CANCER GENE THER 1998 JUL-AUG;5(4):195-206, XP001039608
- PEELLE B, LORENS J, LI W, BOGENBERGER J, PAYAN DG, ANDERSON DC.: "Intracellular protein scaffold-mediated display of random peptide libraries for phenotypic screens in mammalian cells." CHEM BIOL. 2001 MAY;8(5):521-34., XP001033600
- ABEDI M R ET AL: "GREEN FLUORESCENT PROTEIN AS A SCAFFOLD FOR INTRACELLULAR PRESENTATION OF PEPTIDES" NUCLEIC ACIDS RESEARCH, IRL PRESS LTD., OXFORD, GB, vol. 26, no. 2, 1998, pages 623-630, XP001036725 ISSN: 0305-1048
- PEELLE B, GURURAJA TL, PAYAN DG, ANDERSON DC: "Characterization and use of green fluorescent proteins from Renilla mulleri and Ptilosarcus guernyi for the human cell display of functional peptides." J PROTEIN CHEM. 2001 AUG;20(6):507-19., XP008000409

## Description

### FIELD OF THE INVENTION

The invention relates to methods and compositions utilizing Renilla green fluorescent proteins (GFP). In particular, the invention relates to the use of Renilla GFP proteins as reporters for cell assays, particularly intracellular assays, including methods of screening libraries using GFP.

### BACKGROUND OF THE INVENTION

The field of biomolecule screening for biologically and therapeutically relevant compounds is rapidly growing. Relevant biomolecules that have been the focus of such screening include chemical libraries, nucleic acid libraries and peptide libraries, in search of molecules that either inhibit or augment the biological activity of identified target molecules. With particular regard to peptide libraries, the isolation of peptide inhibitors of targets and the identification of formal binding partners of targets has been a key focus. However, one particular problem with peptide libraries is the difficulty assessing whether any particular peptide has been expressed, and at what level, prior to determining whether the peptide has a biological effect.

The green fluorescent protein from *Aequorea Victoria* (termed herein "aGFP') is a 238 amino acid protein. The crystal structure of the protein and of several point mutants has been solved (Ormo et al., Science 273, 1392-5, 1996; Yang et al., Nature Biotechnol. 14, 1246-51. 1996). The fluorophore, consisting of a modified tripeptide, is buried inside a relatively rigid beta-can structure, where it is almost completely protected from solvent access. The fluorescence of this protein is sensitive to a number of point mutations (Phillips, G.N., Curr. Opin. Struct. Biol. 7, 821-27, 1997). The fluorescence appears to be a sensitive indication of the preservation of the native structure of the protein, since any disruption of the structure allowing solvent access to the fluorophoric tripeptide will quench the fluorescence.

A GFP from *Renilla mulleri* (termed herein "rGFP"), has been reported recently: see WO 99/49019, hereby expressly incorporated by reference.

It is an object of the present invention to provide methods and compositions comprising rGFP.

### SUMMARY

In accordance with the above objects, the present invention provides retroviral vectors comprising a nucleic acid encoding a Renilla GFP. These vectors can further comprise an IRES site.

In an additional aspect, the invention provides a cell comprising a retroviral vector of the invention.

In a further aspect, the present invention provides libraries of retroviral vectors wherein each vector comprises a nucleic acid encoding a Renilla GFP fused to a protein of interest.

In a further aspect the invention provides a library of cells comprising a library of retroviral vectors of the invention.

### DETAILED DESCRIPTION OF THE FIGURES

Figure 1 depicts a homology lineup between the GFPs of *Renilla Mulleri, Pitilosarcus Gumeyi, Aequorea* and its enhanced version, EGFP. The underlined residues are the fluorescent tripeptide (chromophore). Identity, strong similarity and weak similarity are depicted.
Figure 2 depicts the nucleic acid sequence of the wild type rGFP.
Figure 3 depicts the nucleic acid sequence of the wild type pGFP.

### DETAILED DESCRIPTION OF THE INVENTION

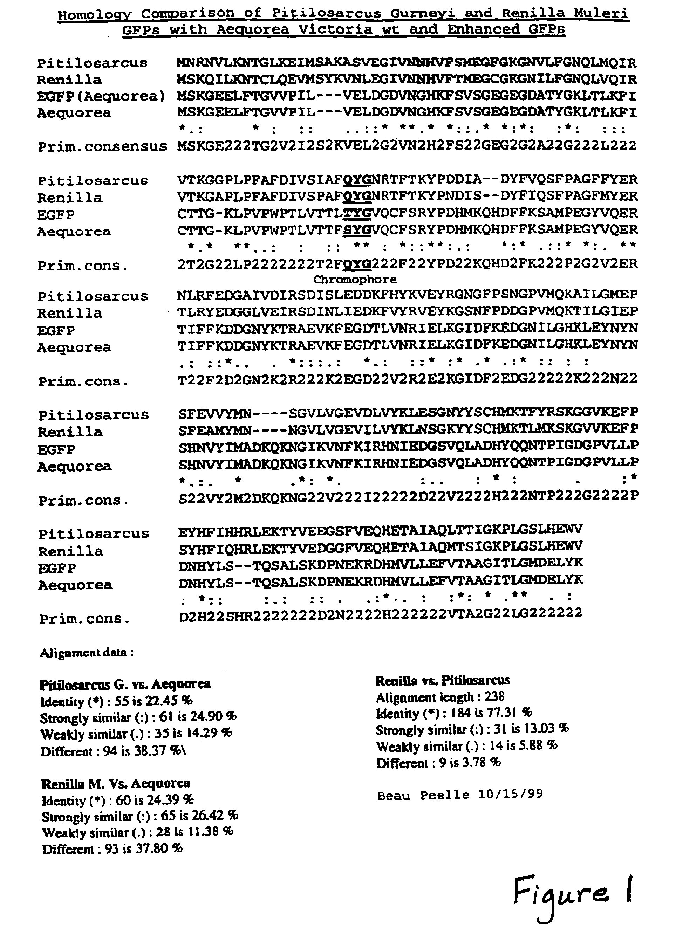

The present invention is directed to the use of Renilla green fluorescent protein (hereinafter "rGFP"), in a variety of methods and compositions that exploit the autofluorescent properties of rGFP. These methods include, but are not limited to, the use of rGFP as a reporter molecule in cell screening assays, including intracellular assays; the use of rGFP as a scaffold protein for fusions with random peptide libraries; etc. Similarly, compositions of rGFP are provided, including constructs of rGFP such as fusion constructs that include rGFP as a reporter gene, retroviral constructs including rGFP and internal ribosome entry sites (IRES), etc. Basically, the invention provides a number of novel uses for rGFP, similar to those outlined for aGFP in WO 95/07463, hereby incorporated by reference in its entirety. The amino acid sequence of Pitilosarcus Gurneyi green fluorescent protein ("pGFP") shown in Figure 1 and is also depicted in WO 99/49019.

In a preferred embodiment, the invention provides compositions including rGFP. By "Renilla green fluorescent protein" or "rGFP" herein is meant a protein that has significant homology, as defined herein, to the wild-type protein of Figure 1, as depicted in WO 99/49019, hereby incorporated by reference in its entirety.

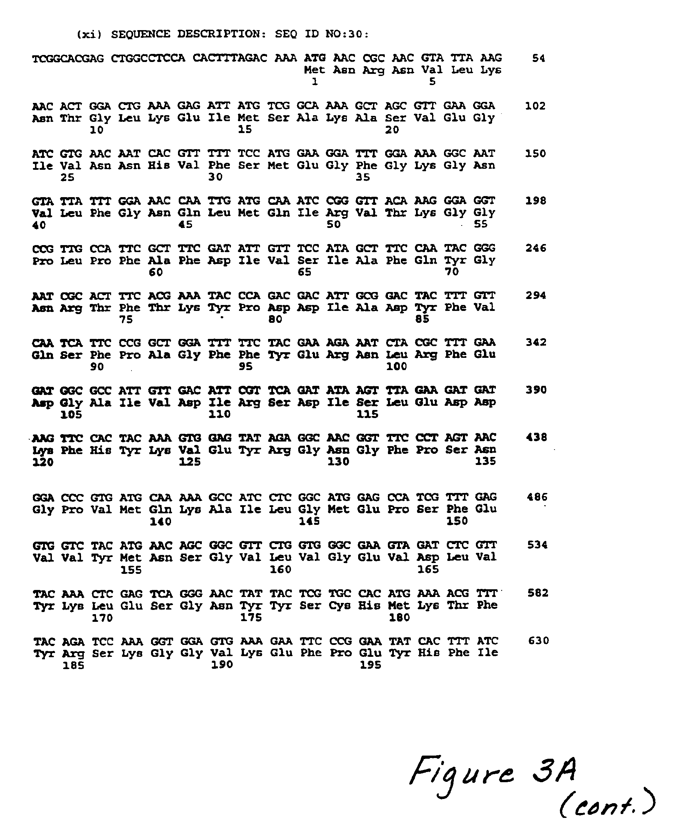

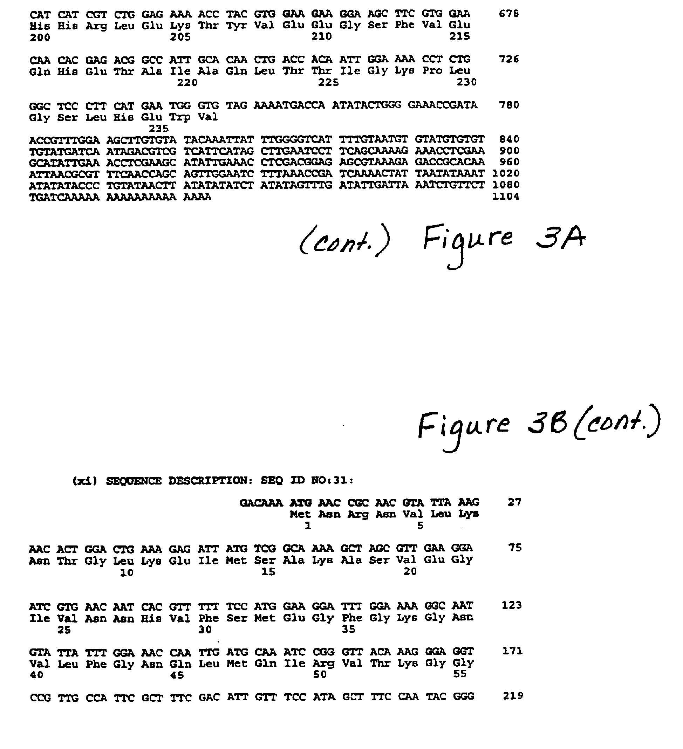

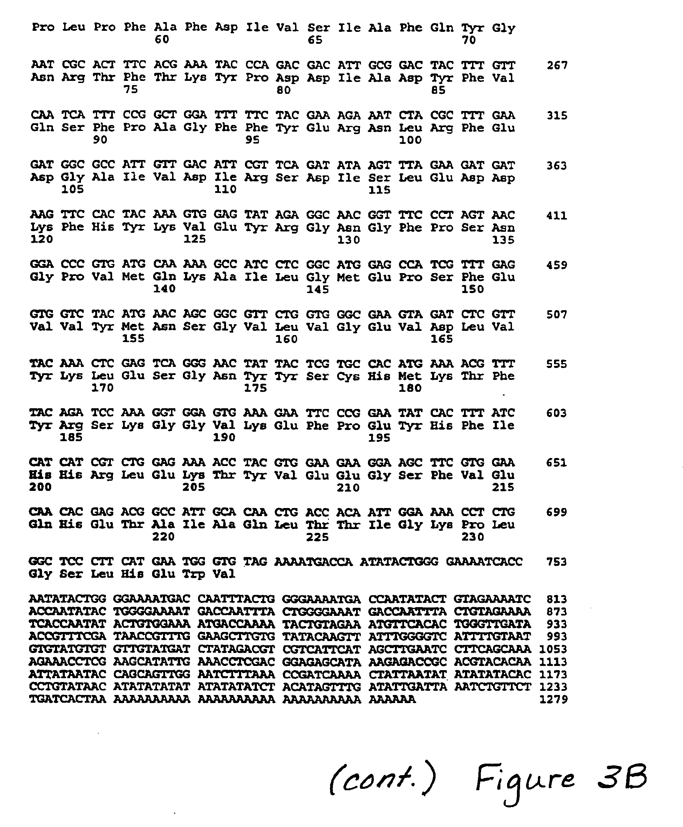

By Ptilosarcus green fluorescent protein" or "pGFP" herein is meant a protein that has significant homology, as defined herein, to the wild-type protein of Figure 3. as depicted in WO 99/49019. hereby incorporated by reference in its entirety.

An rGFP protein of the present invention may be identified in several ways. "Protein" in this sense includes proteins, polypeptides, and peptides. A rGFP nucleic acid or protein is initially identified by substantial nucleic acid and/or amino acid sequence homology to the sequences shown in Figures 1, 2 and 3. Such homology can be based upon the overall nucleic acid or amino acid sequence.

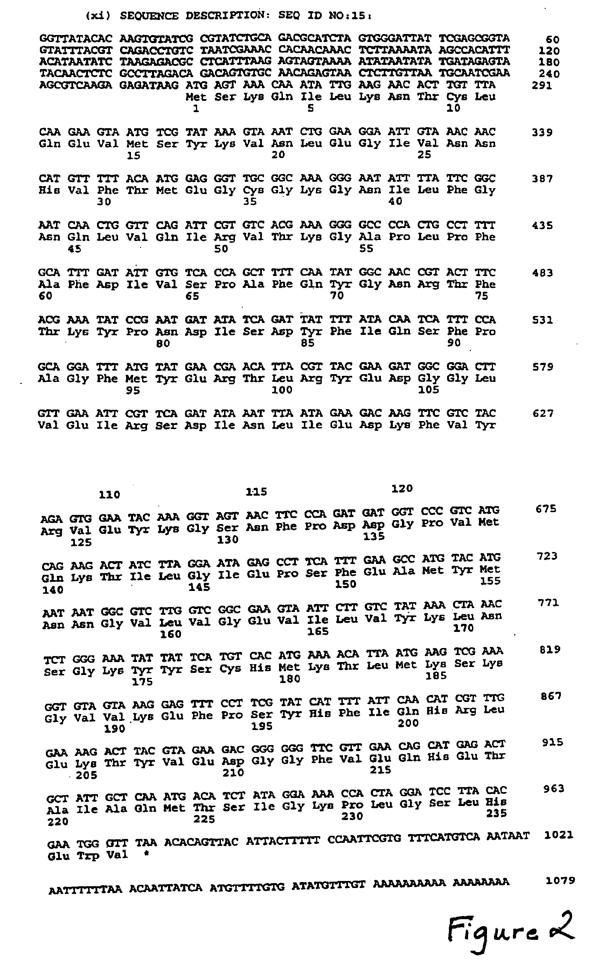

As used herein, a protein is a "rGFP protein" or "pGFP" if the overall homology of the protein sequence to the amino acid sequence shown in Figures 2 or 3 is preferably greater than about 75%, more preferably greater than about 80%, even more preferably greater than about 85% and most preferably greater than 90%. In some embodiments the homology will be as high as about 93 to 95 or 98%.

Homology in this context means sequence similarity or identity, with identity being preferred. This homology will be determined using standard techniques known in the art, including, but not limited to, the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc Natl. Acad. Sci. U.S.A. 85:2444 (1988), by computerized implementations of these algorithms (GAP, BESTFIT, FASTA, and TFASTA in the Wisconsin Genetics Software Package, Genetics Computer Group, 575 Science Drive, Madison, WI), or the Best Fit sequence program described by Devereux et al., Nucl. Acid Res. 12:387-95 (1984), preferably using the default settings, or by inspection.

In a preferred embodiment, similarity is calculated by FastDB based upon the following parameters: mismatch penalty of 1.0; gap size penalty of 0.33, joining penalty of 30.0 ("Current methods in Comparison and Analysis", Macromolecule Sequencing and Synthesis, selected methods and Applications, pp. 127-149 (1998), Alan R. Liss, Inc.). Another example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle, J. Mol. Evol. 35:351-60 (1987); the method is similar to that described by Higgins and Sharp CABIOS 5:151-3 (1989). Useful PILEUP parameters including a default gap weight of 3.00, a default gap length weight of 0.10, and weighted end gaps.

An additional example of a useful algorithm is the BLAST algorithm, described in Altschul et al., J. Mol. Biol. 215: 403-410 (1990) and Karlin et al., Proc. Natl. Acad. Sci. U.S.A. 90:5873-87 (1993). A particularly useful BLAST program is the WU-BLAST-2 program which was obtained from Altschul et al., Methods in Enzymology 266:460-480 (1996); http://blast.wustl/edu/blast/ README.html]. WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched; however, the values may be adjusted to increase sensitivity A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

In a similar manner, "percent (%) nucleic acid sequence identity" with respect to the coding sequence of the polypeptides identified herein is defined as the percentage of nucleotide residues in a candidate sequence that are identical with the nucleotide residues in the coding sequence of the rGFP proteins (see Figure 1). A preferred method utilizes the BLASTN module of WU-BLAST-2 set to the default parameters, with overlap span and overlap fraction set to 1 and 0.125, respectively.

An additional useful algorithm is gapped BLAST as reported by Altschul et al., Nucl. Acid Res. 25:3389-3402 (1997). Gapped BLAST uses BLOSUM-62 substitution scores; threshold T parameter set to 9; the two-hit method to trigger ungapped extensions; charges gap lengths of k a cost of 10+*k*; *X*ᵤ set to 16, and *X*_{g} set to 40 for database search stage and to 67 for the output stage of the algorithms. Gapped alignments are triggered by a score corresponding to -22 bits.

The alignment may include the introduction of gaps in the sequences to be aligned. In addition, for sequences which contain either more or fewer amino acids than the protein sequences shown in Figure 1, it is understood that the percentage of homology will be determined based on the number of homologous amino acids in relation to the total number of amino acids. Thus, for example, homology of sequences shorter than that shown in Figure 1, as discussed below, will be determined using the number of amino acids in the shorter sequence.

GFP proteins of the present invention may be shorter or longer than the amino acid sequences shown in Figure 1. Thus, in a preferred embodiment, included within the definition of GFP proteins are portions or fragments of the sequences depicted herein. Portions or fragments of r- and pGFP proteins are considered GFP proteins if a) they share at least one antigenic epitope; or b) have at least the indicated homology; c) preferably have GFP biological activity, e.g., including, but not limited to, autofluorescence; or d) fold into a stable structure that is similar to the wild-type structure.

For example, r- and pGFP deletion mutants can be made. At the N-terminus, it is known that only the first amino acid of the aGFP protein may be deleted without loss of fluorescence. At the C-terminus of the aGFP, up to 7 residues can be deleted without loss of fluorescence; see Phillips et al., Current Opin. Structural Biol. 7:821 (1997)). This presumably applies to rGFP as well.

In one embodiment, the rGFP proteins are derivative or variant GFP proteins. That is, as outlined more fully below, the derivative GFP will contain at least one amino acid substitution, deletion or insertion, with amino acid substitutions being particularly preferred. The amino acid substitution, insertion or deletion may occur at any residue within the GFP protein. These variants ordinarily are prepared by site specific mutagenesis of nucleotides in the DNA encoding the GFP protein, using cassette or PCR mutagenesis or other techniques well known in the art, to produce DNA encoding the variant, and thereafter expressing the DNA in recombinant cell culture as is known in the art and outlined herein. However, variant GFP protein fragments having up to about 100-150 residues may be prepared by *in vitro* synthesis using established techniques. Amino acid sequence variants are characterized by the predetermined nature of the variation, a feature that sets them apart from naturally occurring allelic or interspecies variation of the GFP protein amino acid sequence. The variants typically exhibit the same qualitative biological activity as the naturally occurring analogue, although variants can also be selected which have modified characteristics as will be more fully outlined below. That is, in a preferred embodiment, when non-wild-type GFP is used, the derivative preferably has at least 1% of wild-type fluorescence, with at least about 10% being preferred, at least about 50-60% being particularly preferred and 95% to 98% to 100% being especially preferred. In general, what is important is that there is enough fluorescence to allow sorting and/or detection above background, for example using a fluorescence-activated cell sorter (FACS) machine. However, in some embodiments, for example when fusion proteins with GFP are made, it is possible to detect the fusion proteins non-fluorescently, using, for example, antibodies directed to either an epitope tag (i.e. purification sequence) or to the GFP itself. In this case the GFP scaffold does not have to be fluorescent, if it can be shown that the GFP is folding correctly and/or reproducibly.

Thus, the rGFP or pGFP may be wild type or variants thereof. These variants fall into one or more of three classes: substitutional, insertional or deletional variants. These variants ordinarily are prepared by site specific mutagenesis of nucleotides in the DNA encoding the GFP, using cassette or PCR mutagenesis or other techniques well known in the art, to produce DNA encoding the variant, and thereafter expressing the DNA in recombinant cell culture as outlined herein. However, variant protein fragments having up to about 100-150 residues may be prepared by in vitro synthesis using established techniques. Amino acid sequence variants are characterized by the predetermined nature of the variation, a feature that sets them apart from naturally occurring allelic or interspecies variation of the rGFP amino acid sequence. The variants typically exhibit the same qualitative biological activity as the naturally occurring analogue, although variants can also be selected which have modified characteristics as will be more fully outlined below.

While the site or region for introducing an amino acid sequence variation is predetermined, the mutation per se need not be predetermined. For example, in order to optimize the performance of a mutation at a given site, random mutagenesis may be conducted at the target codon or region and the expressed scaffold variants screened for the optimal combination of desired activity. Techniques for making substitution mutations at predetermined sites in DNA having a known sequence are well known, for example, M13 primer mutagenesis and PCR mutagenesis. Screening of the mutants is done using assays of scaffold protein activities.

Amino acid substitutions are typically of single residues; insertions usually will be on the order of from about 1 to 20 amino acids, although considerably larger insertions may be tolerated. Deletions range from about 1 to about 20 residues, although in some cases deletions may be much larger.

Substitutions, deletions, insertions or any combination thereof may be used to arrive at a final derivative. Generally these changes are done on a few amino acids to minimize the alteration of the molecule. However, larger changes may be tolerated in certain circumstances. When small alterations in the characteristics of the p- or rGFP protein are desired, substitutions are generally made in accordance with the following chart:

| | Chart I |
|---|---|
| Original Residue | Exemplary Substitutions |
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

Substantial changes in function or immunological identity are made by selecting substitutions that are less conservative than those shown in Chart I. For example, substitutions may be made which more significantly affect: the structure of the polypeptide backbone in the area of the alteration, for example the alpha-helical or beta-sheet structure; the charge or hydrophobicity of the molecule at the target site; or the bulk of the side chain. The substitutions which in general are expected to produce the greatest changes in the polypeptide's properties are those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g. lysyl, arginyl, or histidyl, is substituted for (or by) an electronegative residue, e.g. glutamyl or aspartyl; or (d) a residue having a bulky side chain, e.g. phenylalanine, is substituted for (or by) one not having a side chain, e.g. glycine.

As outlined above, the variants typically exhibit the same qualitative biological activity (i.e. fluorescence) although variants also are selected to modify the characteristics of the GFP protein as needed.

In a preferred embodiment specific residues of rGFP are substituted, resulting in proteins with modified characteristics. Such substitutions may occur at one or more residues, with 1-10 substitutions being preferred. Preferred characteristics to be modified include range of spectral emission, including shifts in peak emission, rate of folding, stability, expression levels, toxicity, and emission intensity. As is known in the art, there are a number of aGFP variants with desirable properties, and these may be varied in rGFP and pGFP as well.

In a preferred embodiment, residue 46 of rGFP (corresponding to residue 43 of aGFP) is substituted with a Thr or an Ala.

In a preferred embodiment, residue 69 of rGFP (corresponding to residue 65 of aGFP) is substituted with a Thr, Ile, Cys, Ser, Leu, Ala or Gly.

In a preferred embodiment, residue 70 of rGFP (corresponding to residue 66 of aGFP) is substituted with an His, Phe, or Trp.

In a preferred embodiment, residue 72 of rGFP (corresponding to residue 68 of aGFP) is substituted with a Val or Leu.

In a preferred embodiment, residue 76 of rGFP (corresponding to residue 72 of aGFP) is substituted with an Ser or Ala.

In a preferred embodiment, residue 101 of rGFP (corresponding to residue 99 of aGFP) is substituted with an Phe or Ser.

In a preferred embodiment, residue 125 of rGFP (corresponding to residue 123 of aGFP) is substituted with an Ile.

In a preferred embodiment, residue 147 of rGFP (corresponding to residue 145 of aGFP) is substituted with a Tyr, Phe or His.

In a preferred embodiment, residue 148 of rGFP (corresponding to residue 146 of aGFP) is substituted with an N or I.

preferred embodiment, residue 150 of rGFP (corresponding to residue 148 of aGFP) is substituted with an His or Arg.

In a preferred embodiment, residue 155 of rGFP (corresponding to residue 153 of aGFP) is substituted with a Thr or Ala.

In a preferred embodiment, residue 162 of rGFP (corresponding to residue 163 of aGFP) is substituted with a Val or Ala.

In a preferred embodiment, residue 166 of rGFP (corresponding to residue 167 of aGFP) is substituted with an Ile or Thr.

In a preferred embodiment, residue 200 of rGFP (corresponding to residue 202 of aGFP) is substituted with an Ser or Phe.

In a preferred embodiment, residue 201 of rGFP (corresponding to residue 203 of aGFP) is substituted with an Ile or Thr.

In a preferred embodiment, residue 203 of rGFP (corresponding to residue 205 of aGFP) is substituted with an Ser or Thr.

In a preferred embodiment, residue 210 of rGFP (corresponding to residue 212 of aGFP) is substituted with an N or Val.

In addition, rGFP proteins can be made that are longer than the wild-type, for example, by the addition of epitope or purification tags, the addition of other fusion sequences, etc., as is more fully outlined below.

In a preferred embodiment, the rGFP protein is fused to a protein of interest. This may be done, for example, to allowing tracking or localization of the protein of interest to a particular subcellular location, or to allow for quantification of expression, etc.

In a preferred embodiment, the rGFP is fused to a random peptide to form a fusion polypeptide. By "fused" or "operably linked" herein is meant that the random peptide, as defined below, and the GFP protein are linked together, in such a manner as to minimize the disruption to the stability of the GFP structure (i.e. it can retain biological activity). That is, the GFP preferably retains its ability to fluoresce, or maintains a Tm of at least 42°C. As outlined below, the fusion polypeptide (or fusion polynucleotide encoding the fusion polypeptide) can comprise further components as well, including multiple peptides at multiple loops, fusion partners, etc.

The fusion polypeptide preferably includes additional components, including, but not limited to, fusion partners and linkers.

In a preferred embodiment, the random peptide is fused to the N-terminus of the rGFP. The fusion can be direct, i.e. with no additional residues between the C-terminus of the peptide and the N-terminus of the rGFP, or indirect; that is, intervening amino acids are used, such as one or more fusion partners, including a linker. In this embodiment, preferably a presentation structure is used, to confer some conformational stability to the peptide. Particularly preferred embodiments include the use of dimerization sequences.

In one embodiment, N-terminal residues of the rGFP are deleted, i.e. one or more amino acids of the p- or rGFP can be deleted and replaced with the peptide. However, as noted above, deletions of more than 7 amino acids may render the rGFP less fluorescent, and thus larger deletions are generally not preferred. In a preferred embodiment, the fusion is directly to the first amino acid of the rGFP.

In a preferred embodiment, the random peptide is fused to the C-terminus of the rGFP. As above for N-terminal fusions, the fusion can be direct or indirect, and C-terminal residues may be deleted.

In a preferred embodiment, peptides and fusion partners are added to both the N- and the C-terminus of the rGFP. As the N- and C-terminus of rGFP are putatively on the same "face" of the protein as is the case for aGFP, in spatial proximity (within 18 Å), it is possible to make a noncovalently "circular" rGFP protein using the components of the invention. Thus for example, the use of dimerization sequences can allow a noncovalently cyclized protein; by attaching a first dimerization sequence to either the N- or C-terminus of p- or rGFP, and adding a random peptide and a second dimerization sequence to the other terminus, a large compact structure can be formed.

In a preferred embodiment, the random peptide is fused to an internal position of the rGFP that is, the peptide is inserted at an internal position of the rGFP. While the peptide can be inserted at virtually any position, preferred positions include insertion at the very tips of "loops" on the surface of the rGFP, to minimize disruption of the rGFP beta-can protein structure.

In a preferred embodiment, the random peptide is inserted in rGFP loops. That is, libraries of random peptides (or, alternatively single peptides) can be inserted into or replace external loops. In a preferred embodiment, the loop comprises rGFP residues from about 103 to about 106. As outlined below, this can be either an insertion (e.g. without replacing any residues), or the addition of the random peptides or other fusion partners results in the replacement of one or more of the native residues. Similar preferred embodiments utilize replacements or insertions at positions from about 117 to about 120 of both rGFP; replacements or insertions at positions from about 157 to about 158; replacements or insertions at positions from about 170 to about 173; replacements or insertions at positions from about 186 to about 191; or replacements or insertions at positions from about 208 to about 213. More preferably the insertion or replacement will take place between residues 117-120, 170-173 or 208-213. Most preferably the insertion will take place between residues 170-173 or 208-213.

In a preferred embodiment, the random peptide is inserted, without any deletion of rGFP residues. That is, the insertion point is between two amino acids in the loop, adding the new amino acids of the peptide and fusion partners, including linkers. Generally, when linkers are used, the linkers are directly fused to the rGFP, with additional fusion partners, if present, being fused to the linkers and the peptides.

In a preferred embodiment, the peptide is inserted into the rGFP, with one or more rGFP residues being deleted; that is, the random peptide (and fusion partners, including linkers) replaces one or more residues. In general, when linkers are used, the linkers are attached directly to the rGFP, thus it is linker residues which replace the rGFP residues, again generally at the tip of the loop. In general, when residues are replaced, from one to five residues of rGFP are deleted, with deletions of one, two, three, four and five amino acids all possible.

In a preferred embodiment, peptides (including fusion partners, if applicable) can be inserted into more than one loop of the scaffold at a time. Thus, for example, adding peptides to two loops can increase the complexity of the library but still allow presentation of these loops on the same face of the protein. Similarly, it is possible to add peptides to one or more loops and add other fusion partners to other loops, such as targeting sequences, etc.

Thus, fusion polypeptides comprising rGFP and random peptides are provided. Similarly, the invention provides fusion nucleic acids encoding the fusion polypeptides. In addition, to facilitate the introduction of random peptides into the rGFP, a preferred embodiment provides rGFP nucleic acids with a multisite cloning site inserted into at least one loop outlined above.

In a preferred embodiment, the fusion polypeptides further comprise fusion partners. By "fusion partner' herein is meant a sequence that is associated with the random peptide that confers upon all members of the library in that class a common function or ability. Fusion partners can be heterologous (i.e. not native to the host cell), or synthetic (not native to any cell). Suitable fusion partners include, but are not limited to: a) presentation structures, as defined below, which provide the peptides in a conformationally restricted or stable form; b) targeting sequences, defined below, which allow the localization of the peptide into a subcellular or extracellular compartment; c) rescue sequences as defined below, which allow the purification or isolation of either the peptides or the nucleic acids encoding them; d) stability sequences, which confer stability or protection from degradation to the peptide or the nucleic acid encoding it, for example resistance to proteolytic degradation; e) linker sequences, which conformationally decouple the random peptide elements from the scaffold itself, which keep the peptide from interfering with scaffold folding; or f), any combination of a), b), c), d) and e) as well as linker sequences as needed.

In a preferred embodiment; the fusion partner is a presentation structure. By "presentation structure" or grammatical equivalents herein is meant a sequence, which, when fused to peptides, causes the peptides to assume a conformationally restricted form. Proteins interact with each other largely through conformationally constrained domains. Although small peptides with freely rotating amino and carboxyl termini can have potent functions as is known in the art, the conversion of such peptide structures into pharmacologic agents is difficult due to the inability to predict side-chain positions for peptidomimetic synthesis. Therefore the presentation of peptides in conformationally constrained structures will benefit both the later generation of pharmacophore models and pharmaceuticals and will also likely lead to higher affinity interactions of the peptide with the target protein. This fact has been recognized in the combinatorial library generation systems using biologically generated short peptides in bacterial phage systems. A number of workers have constructed small domain molecules in which one might present randomized peptide structures.

Thus, synthetic presentation structures, i.e. artificial polypeptides, are capable of presenting a randomized peptide as a conformationally-restricted domain. Generally such presentation structures comprise a first portion joined to the N-terminal end of the randomized peptide, and a second portion joined to the C-terminal end of the peptide; that is, the peptide is inserted into the presentation structure, although variations may be made, as outlined below, in which elements of the presentation structure are included within the random peptide sequence. To increase the functional isolation of the randomized expression product, the presentation structures are selected or designed to have minimal biologically activity when expressed in the target cell.

Preferred presentation structures maximize accessibility to the peptide by presenting it on an exterior surface such as a loop, and also cause further conformational constraints in a peptide. Accordingly, suitable presentation structures include, but are not limited to, dim sequences, minibody structures, loops on β-turns and coiled-coil stem structures in which residues not critical to structure are randomized, zinc-finger domains, cysteine-linked (disulfide) structures, transglutaminase linked structures, cyclic peptides, B-loop structures, helical barrels or bundles, leucine zipper motifs, etc.

In a preferred embodiment, the presentation structure is a coiled-coil structure, allowing the presentation of the randomized peptide on an exterior loop. See, for example, Myszka et al., Biochem. 33:2362-2373 (1994), hereby incorporated by reference). Using this system investigators have isolated peptides capable of high affinity interaction with the appropriate target. In general, coiled-coil structures allow for between 6 to 20 randomized positions.

### A preferred coiled-coil presentation structure is as follows:

MGCAALESEVSALESEVASLESEVAAL**GRGDM**PLAAVKSKLSAVKSKLASVKSKLAACGPP. The underlined regions represent a coiled-coil leucine zipper region defined previously (see Martin et al., EMBO J. 13(22):5303-5309 (1994), incorporated by reference). The bolded GRGDMP region represents the loop structure and when appropriately replaced with randomized peptides (i.e. peptides, generally depicted herein as (X)ₙ, where X is an amino acid residue and n is an integer of at least 5 or 6) can be of variable length. The replacement of the bolded region is facilitated by encoding restriction endonuclease sites in the underlined regions, which allows the direct incorporation of randomized oligonucleotides at these positions. For example, a preferred embodiment generates a Xhol site at the double underlined LE site and a HindIII site at the double-underlined KL site.

In a preferred embodiment, the presentation structure is a minibody structure. A "minibody" is essentially composed of a minimal antibody complementarity region. The minibody presentation structure generally provides two randomizing regions that in the folded protein are presented along a single face of the tertiary structure. See for example Bianchi et al., J. Mol. Biol. 236(2):649-59 (1994), and references cited therein, all of which are incorporated by reference). Investigators have shown this minimal domain is stable in solution and have used phage selection systems in combinatorial libraries to select minibodies with peptide regions exhibiting high affinity, Kd = 10⁻⁷, for the pro-inflammatory cytokine IL-6.

A preferred minibody presentation structure is as follows:
MGRNSQATS**GFT***F***SHF**YMEWVRGGEYIAASR**HKHNKY**TTEYSASVKGRYIVSRDTSQSILYLQKKKG
PP. The bold, underline regions are the regions which may be randomized. The italized phenylalanine must be invariant in the first randomizing region. The entire peptide is cloned in a three-oligonucleotide variation of the coiled-coil embodiment, thus allowing two different randomizing regions to be incorporated simultaneously. This embodiment utilizes non-palindromic BstXl sites on the termini.

In a preferred embodiment, the presentation structure is a sequence that contains generally two cysteine residues, such that a disulfide bond may be formed, resulting in a conformationally constrained sequence. This embodiment is particularly preferred ex vivo, for example when secretory targeting sequences are used. As will be appreciated by those in the art, any number of random sequences, with or without spacer or linking sequences, may be flanked with cysteine residues. In other embodiments, effective presentation structures may be generated by the random regions themselves. For example, the random regions may be "doped" with cysteine residues which, under the appropriate redox conditions, may result in highly crosslinked structured conformations, similar to a presentation structure. Similarly, the randomization regions may be controlled to contain a certain number of residues to confer β-sheet or α-helical structures.

In a preferred embodiment, the presentation sequence confers the ability to bind metal ions to confer secondary structure. Thus, for example, C2H2 zinc finger sequences are used; C2H2 sequences have two cysteines and two histidines placed such that a zinc ion is chelated. Zinc finger domains are known to occur independently in multiple zinc-finger peptides to form structurally independent, flexibly linked domains. See J. Mol. Biol. 228:619 (1992). A general consensus sequence is (5 amino acids)-C-(2 to 3 amino acids)-C-(4 to 12 amino acids)-H-(3 amino acids)-H-(5 amino acids). A preferred example would be -FQCEEC-random peptide of 3 to 20 amino acids-HIRSHTG-.

Similarly, CCHC boxes can be used (see Biochem. Biophys. Res. Commun. 242:385 (1998)), that have a consensus seqeunce -C-(2 amino acids)-C-(4 to 20 random peptide)-H-(4 amino acids)-C-(see Bavoso et al., Biochem. Biophys. Res. Comm. 242(2):385 (1998), hereby incorporated by reference. Preferred examples include (1) -VKCFNC-4 to 20 random amino acids-HTARNCR-, based on the nucleocapsid protein P2; (2) a sequence modified from tehat of the naturally occuring zinc-binding peptide of the Lasp-1 LIM domain (Hammarstrom et al., Biochem. 35:12723 (1996)); and (3) -MNPNCARCG-4 to 20 random amino acids-HKACF-, based on the nmr structural ensemble 1ZFP (Hammarstrom et al., Biochem. 35 U.S.C. 35(39):12723 (1996).

In a preferred embodiment, the presentation structure is a dimerization sequence, including self-binding peptides. A dimerization sequence allows the non-covalent association of two peptide sequences, which can be the same or different, with sufficient affinity to remain associated under normal physiological conditions. These sequences may be used in several ways. In a preferred embodiment, one terminus of the random peptide is joined to a first dimerization sequence and the other terminus is joined to a second dimerization sequence, which can be the same or different from the first sequence. This allows the formation of a loop upon association of the dimerizing sequences. Alternatively, the use of these sequences effectively allows small libraries of random peptides (for example, 10⁴) to become large libraries if two peptides per cell are generated which then dimerize, to form an effective library of 10⁸ (10⁴ X 10⁴). It also allows the formation of longer random peptides, if needed, or more structurally complex random peptide molecules. The dimers may be homo- or heterodimers.

Dimerization sequences may be a single sequence that self-aggregates, or two different sequences that associate. That is, nucleic acids encoding both a first random peptide with dimerization sequence 1, and a second random peptide with dimerization sequence 2, such that upon introduction into a cell and expression of the nucleic acid, dimerization sequence 1 associates with dimerization sequence 2 to form a new random peptide structure. The use of dimerization sequences allows the "circularization" of the random peptides; that is, if a dimerization sequence is used at each terminus of the peptide, the resulting structure can form a "stem-loop" type of structure. Furthermore, the use of dimerizing sequences fused to both the N- and C-terminus of the scaffold such as GFP forms a noncovalently cyclized scaffold random peptide library.

Suitable dimerization sequences will encompass a wide variety of sequences. Any number of protein-protein interaction sites are known. In addition, dimerization sequences may also be elucidated using standard methods such as the yeast two hybrid system, traditional biochemical affinity binding studies, or even using the present methods. See U.S.S.N. 60/080,444, filed April 2, 1998, hereby incorporated by reference in its entireity. Particularly preferred dimerization peptide sequences include, but are not limited to, -EFLIVKS-, EEFLIVKKS-, -FESIKLV-, and -VSIKFEL-.

In a preferred embodiment, the fusion partner is a targeting sequence. As will be appreciated by those in the art, the localization of proteins within a cell is a simple method for increasing effective concentration and determining function. For example, RAF1 when localized to the mitochondrial membrane can inhibit the anti-apoptotic effect of BCL-2. Similarly, membrane bound Sos induces Ras mediated signaling in T-lymphocytes. These mechanisms are thought to rely on the principle of limiting the search space for ligands, that is to say, the localization of a protein to the plasma membrane limits the search for its ligand to that limited dimensional space near the membrane as opposed to the three dimensional space of the cytoplasm. Alternatively, the concentration of a protein can also be simply increased by nature of the localization. Shuttling the proteins into the nucleus confines them to a smaller space thereby increasing concentration. Finally, the ligand or target may simply be localized to a specific compartment, and inhibitors must be localized appropriately.

Thus, suitable targeting sequences include, but are not limited to, binding sequences capable of causing binding of the expression product to a predetermined molecule or class of molecules while retaining bioactivity of the expression product, (for example by using enzyme inhibitor or substrate sequences to target a class of relevant enzymes); sequences signalling selective degradation, of itself or co-bound proteins; and signal sequences capable of constitutively localizing the peptides to a predetermined cellular locale, including a) subcellular locations such as the Golgi, endoplasmic reticulum, nucleus, nucleoli, nuclear membrane, mitochondria, chloroplast, secretory vesicles, lysosome, and cellular membrane; and b) extracellular locations via a secretory signal. Particularly preferred is localization to either subcellular locations or to the outside of the cell via secretion.

In a preferred embodiment, the targeting sequence is a nuclear localization signal (NLS). NLSs are generally short, positively charged (basic) domains that serve to direct the entire protein in which they occur to the cell's nucleus. Numerous NLS amino acid sequences have been reported including single basic NLS's such as that of the SV40 (monkey virus) large T Antigen (Pro Lys Lys Lys Arg Lys Val), Kalderon (1984), et al., Cell, 39:499-509; the human retinoic acid receptor-β nuclear localization signal (ARRRRP); NFκB p50 (EEVQRKRQKL; Ghosh et al., Cell 62:1019 (1990); NFκB p65 (EEKRKRTYE; Nolan et al., Cell 64:961 (1991); and others (see for example Boulikas, J. Cell. Biochem. 55(1):32-58 (1994), hereby incorporated by reference) and double basic NLS's exemplified by that of the Xenopus (African clawed toad) protein, nucleoplasmin (Ala Val Lys Arg Pro Ala Ala Thr Lys Lys Ala Gly Gin Ala Lys Lys Lys Lys Leu Asp), Dingwall, et al., Cell, 30:449-458, 1982 and Dingwall, et al., J. Cell Biol., 107:641-849; 1988). Numerous localization studies have demonstrated that NLSs incorporated in synthetic peptides or grafted onto reporter proteins not normally targeted to the cell nucleus cause these peptides and reporter proteins to be concentrated in the nucleus. See, for example, Dingwall, and Laskey, Ann, Rev. Cell Biol., 2:367-390, 1986; Bonnerot, et al., Proc. Natl. Acad. Sci. USA, 84:6795-6799, 1987; Galileo, et al., Proc. Natl. Acad. Sci. USA, 87:458-462, 1990.

In a preferred embodiment, the targeting sequence is a membrane anchoring signal sequence. This is particularly useful since many parasites and pathogens bind to the membrane, in addition to the fact that many.intracellular events originate at the plasma membrane. Thus, membrane-bound peptide libraries are useful for both the identification of important elements in these processes as well as for the discovery of effective inhibitors. The invention provides methods for presenting the randomized expression product extracellularly or in the cytoplasmic space. For extracellular presentation, a membrane anchoring region is provided at the carboxyl terminus of the peptide presentation structure. The randomized epression product region is expressed on the cell surface and presented to the extracellular space, such that it can bind to other surface molecules (affecting their function) or molecules present in the extracellular medium. The binding of such molecules could confer function on the cells expressing a peptide that binds the molecule. The cytoplasmic region could be neutral or could contain a domain that, when the extracellular randomized expression product region is bound, confers a function on the cells (activation of a kinase, phosphatase, binding of other cellular components to effect function). Similarly, the randomized expression product-containing region could be contained within a cytoplasmic region, and the transmembrane region and extracellular region remain constant or have a defined function.

Membrane-anchoring sequences are well known in the art and are based on the genetic geometry of mammalian transmembrane molecules. Peptides are inserted into the membrane based on a signal sequence (designated herein as ssTM) and require a hydrophobic transmembrane domain (herein TM). The transmembrane proteins are inserted into the membrane such that the regions encoded 5' of the transmembrane domain are extracellular and the sequences 3' become intracellular. Of course, if these transmembrane domains are placed 5' of the variable region, they will serve to anchor it as an intracellular domain, which may be desirable in some embodiments. ssTMs and TMs are known for a wide variety of membrane bound proteins, and these sequences may be used accordingly, either as pairs from a particular protein or with each component being taken from a different protein, or alternatively, the sequences may be synthetic, and derived entirely from consensus as artificial delivery domains.

As will be appreciated by those in the art, membrane-anchoring sequences, including both ssTM and TM, are known for a wide variety of proteins and any of these may be used. Particularly preferred membrane-anchoring sequences include, but are not limited to, those derived from CD8, ICAM-2, IL-8R, CD4 and LFA-1.

Useful sequences include sequences from: 1) class I integral membrane proteins such as IL-2 receptor beta-chain (residues 1-26 are the signal sequence, 241-265 are the transmembrane residues; see Hatakeyama et al., Science 244:551 (1989) and von Heijne et al, Eur. J. Biochem. 174:671 (1988)) and insulin receptor β-chain (residues 1-27 are the signal, 957-959 are the transmembrane domain and 960-1382 are the cytoplasmic domain; see Hatakeyama, supra, and Ebina et al., Cell 40:747 (1985)); 2) class II integral membrane proteins such as neutral endopeptidase (residues 29-51 are the transmembrane domain, 2-28 are the cytoplasmic domain; see Malfroy et al., Biochem. Biophys. Res. Commun. 144:59 (1987)); 3) type III proteins such as human cytochrome P450 NF25 (Hatakeyama, supra); and 4) type IV proteins such as human P-glycoprotein (Hatakeyama, supra). Particularly preferred are CD8 and ICAM-2. For example, the signal sequences from CD8 and ICAM-2 lie at the extreme 5' end of the transcript. These consist of the amino acids 1-32 in the case of CD8 (MASPLTRFLSLNLLLLGESILGSGEAKPQAP; Nakauchi et al., PNAS USA 82:5126 (1985) and 1-21 in the case of ICAM-2 (MSSFGYRTLTVALFTLICCPG; Staunton et al., Nature (London) 339:61 (1989)). These leader sequences deliver the construct to the membrane while the hydrophobic transmembrane domains, placed 3' of the random peptide region, serve to anchor the construct in the membrane. These transmembrane domains are encompassed by amino acids 145-195 from CD8 (PQRPEDCRPRGSVKGTGLDFACDIYIWAPLAGICVALLLSLIITLICYHSR; Nakauchi, supra) and 224-256 from ICAM-2 (MVIIVTVVSVLLSLFVTSVLLCFIFGOHLRQQR; Staunton, supra).

Alternatively, membrane anchoring sequences include the GPI anchor, which results in a covalent bond between the molecule and the lipid bilayer via a glycosyl-phosphatidylinositol bond for example in DAF (PNKGSGTTSGTTRLLSGHTCFTLTGLLGTLVTMGLLT, with the bolded serine the site of the anchor; see Homans et al., Nature 333(6170):269-72 (1988), and Moran et al., J. Biol. Chem. 266:1250 (1991)). In order to do this, the GPI sequence from Thy-1 can be cassetted 3' of the variable region in place of a transmembrane sequence.

Similarly, myristylation sequences can serve as membrane anchoring sequences. It is known that the myristylation of c-src recruits it to the plasma membrane. This is a simple and effective method of membrane localization, given that the first 14 amino acids of the protein are solely responsible for this function: MGSSKSKPKDPSQR (see Cross et al., Mol. Cell. Biol. 4(9):1834 (1984); Spencer et al., Science 262:1019-1024 (1993), both of which are hereby incorporated by reference). This motif has already been shown to be effective in the localization of reporter genes and can be used to anchor the zeta chain of the TCR. This motif is placed 5' of the variable region in order to localize the construct to the plasma membrane. Other modifications such as palmitoylation can be used to anchor constructs in the plasma membrane; for example, palmitoylation sequences from the G protein-coupled receptor kinase GRK6 sequence (LLQRLFSRQDCCGNCSDSEEELPTRL, with the bold cysteines being palmitolyated; Stoffel et al., J. Biol. Chem 269:27791 (1994)); from rhodopsin (KQFRNCMLTSLCCGKNPLGD; Barnstable et al., J. Mol. Neurosci. 5(3):207 (1994)); and the p21 H-ras 1 protein (LNPPDESGPGCMSCKCVLS; Capon et al., Nature 302:33 (1983)).

In a preferred embodiment, the targeting sequence is a lysozomal targeting sequence, including, for example, a lysosomal degradation sequence such as Lamp-2 (KFERQ; Dice, Ann. N.Y. Acad. Sci. 674:58 (1992); or lysosomal membrane sequences from Lamp-1 (*MLIPIAGFFALAGLVLIVLIAYLI*GRKRSHAGYQTI, Uthayakumar et al., Cell. Mol. Biol. Res. 41:405 (1995)) or Lamp-2 (*LVPIAVGAALAGVLILVLLAYFIG*LKHHHAGYEQF, Konecki et la., Biochem. Biophys. Res. Comm. 205:1-5 (1994), both of which show the transmembrane domains in italics and the cytoplasmic targeting signal underlined).

Alternatively, the targeting sequence may be a mitrochondrial localization sequence, including mitochondrial matrix sequences (e.g. yeast alcohol dehydrogenase III;
MLRTSSLFTRRVQPSLFSRNILRLQST; Schatz, Eur. J. Biochem. 165:1-6 (1987)); mitochondrial inner membrane sequences (yeast cytochrome c oxidase subunit IV; MLSLRQSIRFFKPATRTLCSSRYLL;
Schatz, supra); mitochondrial intermembrane space sequences (yeast cytochrome c1;
MFSMLSKRWAQRTLSKSFYSTATGAASKSGKLTQKLVTAGVAAAGITASTLLYADSLTAEAMTA;
Schatz, supra) or mitochondrial outer membrane sequences (yeast 70 kD outer membrane protein;
MKSFITRNKTAILATVAATGTAIGAYYYYNQLQQQQQRGKK; Schatz, supra).

The target sequences may also be endoplasmic reticulum sequences, including the sequences from calreticulin (KDEL; Pelham, Royal Society London Transactions B; 1-10 (1992)) or adenovirus E3/19K protein (LYLSRRSFIDEKKMP; Jackson et al., EMBO J. 9:3153 (1990).

Furthermore, targeting sequences also include peroxisome sequences (for example, the peroxisome matrix sequence from Luciferase; SKL; Keller et al., PNAS USA 4:3264 (1987)); famesylation sequences (for example, P21 H-ras 1; LNPPDESGPGCMSCKCVLS, with the bold cysteine famesylated; Capon, supra); geranylgeranylation sequences (for example, protein rab-5A; LTEPTQPTRNQCCSN, with the bold cysteines geranylgeranylated; Farnsworth, PNAS USA 91:11963 (1994)); or destruction sequences (cyclin B1; RTALGDIGN; Klotzbucher et al., EMBO J. 1:3053 (1996)).

In a preferred embodiment, the targeting sequence is a secretory signal sequence capable of effecting the secretion of the fusion polypeptide. There are a large number of known secretory signal sequences which are placed 5' to the variable peptide region, and are cleaved from the peptide region to effect secretion into the extracellular space. Secretory signal sequences and their transferability to unrelated proteins are well known, e.g., Silhavy, et al. (1985) Microbiol. Rev. 49, 398-418. This is particularly useful to generate a peptide capable of binding to the surface of, or affecting the physiology of, a target cell that is other than the host cell, e.g., the cell infected with the retrovirus. In a preferred approach, a fusion product is configured to contain, in series, secretion signal peptide-presentation structure-randomized expression product region-presentation structure. In this manner, target cells grown in the vicinity of cells caused to express the library of peptides, are bathed in secreted peptide. Target cells exhibiting a physiological change in response to the presence of a peptide, e.g., by the peptide binding to a surface receptor or by being internalized and binding to intracellular targets, and the secreting cells are localized by any of a variety of selection schemes and the peptide causing the effect determined. Exemplary effects include variously that of a designer cytokine (i.e., a stem cell factor capable of causing hematopoietic stem cells to divide and maintain their totipotential), a factor causing cancer cells to undergo spontaneous apoptosis, a factor that binds to the cell surface of target cells and labels them specifically, etc.

Suitable secretory sequences are known, including signals from IL-2 (MYRMQLLSCIALSLALVTNS; Villinger et al., J. Immunol. 155:3946 (1995)), growth hormone (MATGSRTSLLLAFGLLCLPWLQEGSAFPT; Roskam et al., Nucleic Acids Res. 7:30 (1979)); preproinsulin (MALWMRLLPLLALLALWGPDPAAAFVN; Bell et al., Nature 284:26 (1980)); and influenza HA protein (MKAKLLVLLYAFVAGDQI: Sekiwawa et al., PNAS 80:3563)), with cleavage between the non-underlined-underlined junction. A particularly preferred secretory signal sequence is the signal leader sequence from the secreted cytokine IL-4, which comprises the first 24 amino acids of IL-4 as follows: MGLTSQLLPPLFFLLACAGNFVHG.

In a preferred embodiment, the fusion partner is a rescue sequence. A rescue sequence is a sequence which may be used to purify or isolate either the peptide or the nucleic acid encoding it.

Thus, for example, peptide rescue sequences include purification sequences such as the His₆ tag for use with Ni affinity columns and epitope tags for detection, immunoprecipitation or FACS (fluoroscence-activated cell sorting). Suitable epitope tags include myc (for use with the commercially available 9E10 antibody), the BSP biotinylation target sequence of the bacterial enzyme BirA, flu tags, lacZ, GST, and Strep tag I and II.

Alternatively, the rescue sequence may be a unique oligonucleotide sequence which serves as a probe target site to allow the quick and easy isolation of the retroviral construct, via PCR, related techniques, or hybridization.

In a preferred embodiment, the fusion partner is a stability sequence to confer stability to the peptide or the nucleic acid encoding it. Thus, for example, peptides may be stabilized by the incorporation of glycines after the initiation methionine (MG or MGGO), for protection of the peptide to ubiquitination as per Varshavsky's N-End Rule, thus conferring long half-life in the cytoplasm. Similarly, two prolines at the C-terminus impart peptides that are largely resistant to carboxypeptidase action. The presence of two glycines prior to the prolines impart both flexibility and prevent structure initiating events in the diproline to be propagated into the peptide structure. Thus, preferred stability sequences are as follows: MG(X)ₙGGPP, where, X is any amino acid and n is an integer of at least four.

The fusion partners may be placed anywhere (i.e. N-terminal, C-terminal, internal) in the structure as the biology and activity permits. In addition, while the discussion has been directed to the fusion of fusion partners to the peptide portion of the fusion polypeptide, it is also possible to fuse one or more of these fusion partners to the rGFP portion of the fusion polypeptide. Thus, for example, the rGFP may contain a targeting sequence (either N-terminally, C-terminatty, or internally, as described below) at one location, and a rescue sequence in the same place or a different place on the molecule. Thus, any combination of fusion partners and peptides and rGFP proteins may be made.

In a preferred embodiment, the fusion partner includes a linker or tethering sequence. Linker sequences between various targeting sequences (for example, membrane targeting sequences) and the other components of the constructs (such as the randomized peptides) may be desirable to allow the peptides to interact with potential targets unhindered. For example, useful linkers include glycine polymers (G)ₙ, glycine-serine polymers (including, for example, (GS)ₙ, (GSGGS)ₙ and (GGGS)ₙ, where n is an integer of at least one), glycine-alanine polymers, alanine-serine polymers, and other flexible linkers such as the tether for the shaker potassium channel, and a large variety of other flexible linkers, as will be appreciated by those in the art. Glycine and glycine-serine polymers are preferred since both of these amino acids are relatively unstructured, and therefore may be able to serve as a neutral tether between components. Glycine polymers are the most preferred as glycine accesses significantly more phi-psi space than even alanine, and is much less restricted tan residues with longer side chains (see Scheraga, Rev. Computational Chem. III73-142 (1992)). Secondly, serine is hydrophilic and therefore able to solubilize what could be a globular glycine chain. Third, similar chains have been shown to be effective in joining subunits of recombinant proteins such as single chain antibodies.

In a preferred embodiment, the peptide is connected to the rGFP via linkers. That is, while one embodiment utilizes the direct linkage of the peptide to the rGFP or of the peptide and any fusion partners to the rGFP protein, a preferred embodiment utilizes linkers at one or both ends of the peptide. That is, when attached either to the N- or C-terminus, one linker may be used. When the peptide is inserted in an internal position, as is generally outlined below, preferred embodiments utilize at least one linker and preferably two, one at each terminus of the peptide. Linkers are generally preferred in order to conformationally decouple any insertion sequence (i.e. the peptide) from the scaffold structure itself, to minimize local distortions in the scaffold structure that can either destabilize folding intermediates or allow access to rGFP's buried tripeptide fluorophore, which decreases (or eliminates) rGFP's fluorescence due to exposure to exogeneous collisional fluorescence quenchers (see Phillips, Curr. Opin. Structural Biology 7:821 (1997), hereby incorporated by reference in its entireity).

Accordingly, as outlined below, when the peptides are inserted into internal positions in the rGFP protein, preferred embodiments utilize linkers, and preferably (gly)n linkers, where n is 1 or more, with n being two, three, four, five and six, although linkers of 7-10 or more amino adds are also possible. Generally in this embodiment, no amino acids with β-carbons are used in the linkers.

In addition, the fusion partners, including presentation structures, may be modified, randomized, and/or matured to alter the presentation orientation of the randomized expression product. For example, determinants at the base of the loop may be modified to slightly modify the internal loop peptide tertiary structure, which maintaining the randomized amino acid sequence.

In a preferred embodiment, combinations of fusion partners are used. Thus, for example, any number of combinations of presentation structures, targeting sequences, rescue sequences, and stability sequences may be used, with or without linker sequences. As will be appreciated by those in the art, using a base vector that contains a cloning site for receiving random and/or biased libraries, one can cassette in various fusion partners 5' and 3' of the library. In addition, as discussed herein, it is possible to have more than one variable region in a construct, either to together form a new surface or to bring two other molecules together. Similarly, as more fully outlined below, it is possible to have peptides inserted at two or more different loops of the rGFP protein, preferably but not required to be on the same "face" of the" rGFP protein.

The invention further provides fusion nucleic acids encoding the fusion polypeptides of the invention. As will be appreciated by those in the art, due to the degeneracy of the genetic code, an extremely large number of nucleic acids may be made, all of which encode the fusion proteins of the present invention. Thus, having identified a particular amino acid sequence, those skilled in the art could make any number of different nucleic acids, by simply modifying the sequence of one or more codons in a way which does not change the amino acid sequence of the fusion protein.

The present invention has specifically contemplated each and every possible variation of polynucleotide that could be made by selecting combinations based on the possible codon choices, and all such variations are to be considered specifically disclosed and equivalent to the sequences of Figures 2 and 3. Codons are preferably selected to fit the host cell in which the enzyme is being produced; that is, codon usage for yeast is used to express in yeast; codon usage for mammalian cells is used to express in mammalian cells; etc. Selection of codons to maximize expression of proteins in a heterologous host is a known technique.

Using the nucleic adds of the present invention which encode a fusion protein, a variety of expression vectors are made. The expression vectors may be either self-feplicating extrachromosomal vectors or vectors which integrate into a host genome. Generally, these expression vectors include transcriptional and translational regulatory nucleic acid operably linked to the nucleic acid encoding the fusion protein. The term "control sequences' refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice. The transcriptional and translational regulatory nucleic acid will generally be appropriate to the host cell used to express the fusion protein; for example, transcriptional and translational regulatory nucleic acid sequences from *Bacillus* are preferably used to express the fusion protein in *Bacillus.* Numerous types of appropriate expression vectors, and suitable regulatory sequences are known in the art for a variety of host cells.

In general, the transcriptional and translational regulatory sequences may include, but are not limited to, promoter sequences, ribosomal binding sites, transcriptional start and stop sequences, translational start and stop sequences, and enhancer or activator sequences. In a preferred embodiment, the regulatory sequences include a promoter and transcriptional start and stop sequences.

Promoter sequences encode either constitutive or inducible promoters. The promoters may be either naturally occurring promoters or hybrid promoters. Hybrid promoters, which combine elements of more than one promoter, are also known in the art, and are useful in the present invention. In a preferred embodiment, the promoters are strong promoters, allowing high expression in cells, particularly mammalian cells, such as the CMV promoter, particularly in combination with a Tet regulatory element.

In addition, the expression vector may comprise additional elements. For example, the expression vector may have two replication systems, thus allowing it to be maintained in two organisms, for example in mammalian or insect cells for expression and in a procaryotic host for cloning and amplification. Furthermore, for integrating expression vectors, the expression vector contains at least one sequence homologous to the host cell genome, and preferably two homologous sequences which flank the expression construct. The integrating vector may be directed to a specific locus in the host cell by selecting the appropriate homologous sequence for inclusion in the vector. Constructs for integrating vectors are well known in the art.

In addition, in a preferred embodiment, the expression vector contains a selectable marker gene to allow the selection of transformed host cells. Selection genes are well known in the art and will vary with the host cell used.

A preferred expression vector system is a retroviral vector system such as is generally described in PCT/US97/01019 and PCT/US97/01048, both of which are hereby expressly incorporated by reference. Preferred retroviral systems and constructs are also outlined below.

The fusion nucleic acids are introduced into the cells for screening, as is more fully outlined below. By "introduced into"or grammatical equivalents herein is meant that the nucleic acids enter the cells in a manner suitable for subsequent expression of the nucleic acid. The method of introduction is largely dictated by the targeted cell type, discussed below. Exemplary methods include CaPO₄ precipitation, liposome fusion, lipofectin®, electroporation, viral infection, etc. The candidate nucleic acids may stably integrate into the genome of the host cell (for example, with retroviral introduction, outlined below), or may exist either transiently or stably in the cytoplasm (i.e. through the use of traditional plasmids, utilizing standard regulatory sequences, selection markers, etc.). As many pharmaceutically important screens require human or model mammalian cell targets, retroviral vectors capable of transfecting such targets are preferred.

The fusion proteins of the present invention are produced by culturing a host cell transformed with an expression vector containing nucleic acid encoding a fusion protein, under the appropriate conditions to induce or cause expression of the fusion protein. The conditions appropriate for fusion protein expression will vary with the choice of the expression vector and the host cell, and will be easily ascertained by one skilled in the art through routine experimentation. For example, the use of constitutive promoters in the expression vector will require optimizing the growth and proliferation of the host cell, while the use of an inducible promoter requires the appropriate growth conditions for induction. In addition, in some embodiments, the timing of the harvest is important. For example, the baculoviral systems used in insect cell expression are lytic viruses, and thus harvest time selection can be crucial for product yield.

Appropriate host cells include yeast, bacteria, archebacteria, fungi, and insect and animal cells, including mammalian cells. Of particular interest are *Drosophila melangaster* cells, *Saccharomyces cerevisiae* and other yeasts, *E. coli, Bacillus subtilis,* SF9 cells, C129 cells, 293 cells, Neurospora, BHK, CHO, COS, and HeLa cells, fibroblasts, Schwanoma cell lines, immortalized mammalian myeloid and lymphoid cell lines, Jurkat cells, mast cells and other endocrine and exocrine cells, and neuronal cells.

In a preferred embodiment, the fusion proteins are expressed in mammalian cells. Mammalian expression systems are also known in the art, and include retroviral systems. A mammalian promoter is any DNA sequence capable of binding mammalian RNA polymerase and initiating the downstream (3') transcription of a coding sequence for the fusion protein into mRNA. A promoter will have a transcription initiating region, which is usually placed 0proximal to the 5' end of the coding sequence, and a TATA box, using a located 25-30 base pairs upstream of the transcription initiation site. The TATA box is thought to direct RNA polymerase II to begin RNA synthesis at the correct site. A mammalian promoter will also contain an upstream promoter element (enhancer element), typically located within 100 to 200 base pairs upstream of the TATA box. An upstream promoter element determines the rate at which transcription is initiated and can act in either orientation. Of particular use as mammalian promoters are the promoters from mammalian viral genes, since the viral genes are often highly expressed and have a broad host range. Examples include the SV40 early promoter, mouse mammary tumor virus LTR promoter, adenovirus major late promoter, herpes simplex virus promoter, and the CMV promoter.

Typically, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-translational cleavage and polyadenylation. Examples of transcription terminator and polyadenlytion signals include those derived from SV40.

The methods of introducing exogenous nucleic acid into mammalian hosts, as well as other hosts, is well known in the art, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei. As outlined herein, a particularly preferred method utilizes retroviral infection, as outlined in PCT US97/01019, incorporated by reference.

As will be appreciated by those in the art, the type of mammalian cells used in the present invention can vary widely. Basically, any mammalian cells may be used, with mouse, rat, primate and human cells being particularly preferred, although as will be appreciated by those in the art, modifications of the system by pseudotyping allows all eukaryotic cells to be used, preferably higher eukaryotes. As is more fully described below, a screen will be set up such that the cells exhibit a selectable phenotype in the presence of a bioactive peptide. As is more fully described below, cell types implicated in a wide variety of disease conditions are particularly useful, so long as a suitable screen may be designed to allow the selection of cells that exhibit an altered phenotype as a consequence of the presence of a peptide within the cell.

Accordingly, suitable cell types include, but are not limited to, tumor cells of all types (particularly melanoma, myeloid leukemia, carcinomas of the lung, breast, ovaries, colon, kidney, prostate, pancreas and testes), cardiomyocytes, endothelial cells, epithelial cells, lymphocytes (T-cell and B cell) , mast cells, eosinophils, vascular intimal cells, hepatocytes, leukocytes including mononuclear leukocytes, stem cells such as haemopoetic, neural, skin, lung, kidney, liver and myocyte stem cells (for use in screening for differentiation and de-differentiation factors), osteoclasts, chondrocytes and other connective tissue cells, keratinocytes, melanocytes, liver cells, kidney cells, and adipocytes. Suitable cells also include known research cells, including, but not limited to, Jurkat T cells, NIH3T3 cells, CHO, Cos, etc. See the ATCC cell line catalog, hereby expressly incorporated by reference.

In one embodiment, the cells may be additionally genetically engineered, that is, contain exogeneous nucleic acid other than the fusion nucleic acid.

In a preferred embodiment, the fusion proteins are expressed in bacterial systems. Bacterial expression systems are well known in the art.

A suitable bacterial promoter is any nucleic acid sequence capable of binding bacterial RNA polymerase and initiating the downstream (3') transcription of the coding sequence of the fusion protein into mRNA. A bacterial promoter has a transcription initiation region which is usually placed proximal to the 5' end of the coding sequence. This transcription initiation region typically includes an RNA polymerase binding site and a transcription initiation site. Sequences encoding metabolic pathway enzymes provide particularly useful promoter sequences. Examples include promoter sequences derived from sugar metabolizing enzymes, such as galactose, lactose and maltose, and sequences derived from biosynthetic enzymes such as tryptophan. Promoters from bacteriophage may also be used and are known in the art. In addition, synthetic promoters and hybrid promoters are also useful; for example, the *tac* promoter is a hybrid of the *trp* and *lac* promoter sequences. Furthermore, a bacterial promoter can include naturally occurring promoters of non-bacterial origin that have the ability to bind bacterial RNA polymerase and initiate transcription.

In addition to a functioning promoter sequence, an efficient ribosome binding site is desirable. In *E. coli*, the ribosome binding site is called the Shine-Delgamo (SD) sequence and includes an initiation codon and a sequence 3-9 nucleotides in length located 3-11 nucleotides upstream of the initiation codon.

The expression vector may also include a signal peptide sequence that provides for secretion of the fusion protein in bacteria. The signal sequence typically encodes a signal peptide comprised of hydrophobic amino acids which direct the secretion of the protein from the cell, as is well known in the art. The protein is either secreted into the growth media (gram-positive bacteria) or into the periplasmic space, located between the inner and outer membrane of the cell (gram-negative bacteria).

The bacterial expression vector may also include a selectable marker gene to allow for the selection of bacterial strains that have been transformed. Suitable selection genes include genes which render the bacteria resistant to drugs such as ampicillin, chloramphenicol, erythromycin, kanamycin, neomycin and tetracycline. Selectable markers also include biosynthetic genes, such as those in the histidine, tryptophan and leucine biosynthetic pathways.

These components are assembled into expression vectors. Expression vectors for bacteria are well known in the art, and include vectors for *Bacillus subtilis, E. coli, Streptococcus cremoris,* and *Streptococcus lividans,* among others.

The bacterial expression vectors are transformed into bacterial host cells using techniques well known in the art, such as calcium chloride treatment, electroporation, and others.

In one embodiment, fusion proteins are produced in insect cells. Expression vectors for the transformation of insect cells, and in particular, baculovirus-based expression vectors, are well known in the art.

In a preferred embodiment, fusion protein is produced in yeast cells. Yeast expression systems are well known in the art, and include expression vectors for *Saccharomyces cerevisiae, Candida albicans* and C. *maltosa, Hansenula polymorpha, Kluyveromyces fragilis* and K lactis, *Pichia guillerimondii* and *P. pastoris, Schizosaccharomyces pombe,* and *Yarrowia lipolytica.* Preferred promoter sequences for expression in yeast include the inducible GAL 1,10 promoter, the promoters from alcohol dehydrogenase, enolase, glucokinase, gluoose-6-phosphate isomerase, glyceraldehyde-3-phosphate-dehydrogenase, hexokinase, phosphofructokinase, 3-phosphoglycerate mutase, pyruvate kinase, and the acid phosphatase gene. Yeast selectable markers include ADE2, HIS4, LEU2, TRP1, and ALG7, which confers resistance to tunicamycin; the neomycin phosphotransferase gene, which confers resistance to G418; and the CUP1 gene, which allows yeast to grow in the presence of copper ions.

In addition, the fusion polypeptides of the invention may be further fused to other proteins, if desired, for example to increase expression.

In one embodiment, the fusion nucleic acids, proteins and antibodies of the invention are labeled with a label other than the rGFP protein. By "labeled" herein is meant that a compound has at least one element, isotope or chemical compound attached to enable the detection of the compound. In general, labels fall into three classes: a) isotopic labels, which may be radioactive or heavy isotopes: b) immune labels, which may be antibodies or antigens; and c) colored or fluorescent dyes. The labels may be incorporated into the compound at any position.

In a preferred embodiment, the fusion nucleic acids are introduced into the cells to screen for peptides capable of altering the phenotype of a cell.

In a preferred embodiment, a first plurality of cells is screened. That is, the cells into which the fusion nucleic acids are introduced are screened for an altered phenotype. Thus, in this embodiment, the effect of the bioactive peptide is seen in the same cells in which it is made; i.e. an autocrine effect.

By a "plurality of cells" herein is meant roughly from about 10³ cells to 10⁸ or 10⁹, with from 10⁶ to 10⁸ being preferred. This plurality of cells comprises a cellular library, wherein generally each cell within the library contains a member of the peptide molecular library, i.e. a different peptide (or nucleic acid encoding the peptide), although as will be appreciated by those in the art, some cells within the library may not contain a peptide, and some may contain more than species of peptide. When methods other than retroviral infection are used to introduce the candidate nucleic acids into a plurality of cells, the distribution of candidate nucleic acids within the individual cell members of the cellular library may vary widely, as it is generally difficult to control the number of nucleic acids which enter a cell during electroporation, etc. Thus, in a preferred embodiment, libraries of fusion polypeptides comprising p- or rGFP proteins and random peptides are made; that is, a library of random peptides is used to generate a library of fusion polypeptides (and thus a library of fusion polynucleotides encoding the fusion polypeptides).

In a preferred embodiment, the fusion nucleic acids are introduced into a first plurality of cells, and the effect of the peptide is screened in a second or third plurality of cells, different from the first plurality of cells, i.e. generally a different cell type. That is, the effect of the bioactive peptide is due to an extracellular effect on a second cell; i.e. an endocrine or paracrine effect. This is done using standard techniques. The first plurality of cells may be grown in or on one media, and the media is allowed to touch a second plurality of cells, and the effect measured. Alternatively, there may be direct contact between the cells. Thus, "contacting" is functional contact, and includes both direct and indirect. In this embodiment, the first plurality of cells may or may not be screened.

If necessary, the cells are treated to conditions suitable for the expression of the peptide (for example, when inducible promoters are used).

Thus, the methods of the present invention comprise introducing a molecular library of fusion nucleic acids encoding randomized peptides fused to scaffold into a plurality of cells, a cellular library. Each of the nucleic acids comprises a different nucleotide sequence encoding scaffold with a random peptide. The plurality of cells is then screened, as is more fully outlined below, for a cell exhibiting an altered phenotype. The altered phenotype is due to the presence of a bioactive peptide.

By "altered phenotype" or "changed physiology" or other grammatical equivalents herein is meant that the phenotype of the cell is altered in some way, preferably in some detectable and/or measurable way. As will be appreciated in the art, a strength of the present invention is the wide variety of cell types and potential phenotypic changes which may be tested using the present methods. Accordingly, any phenotypic change which may be observed, detected, or measured may be the basis of the screening methods herein. Suitable phenotypic changes include, but are not limited to: gross physical changes such as changes in cell morphology, cell growth, cell viability, adhesion to substrates or other cells, and cellular density; changes in the expression of one or more RNAs, proteins, lipids, hormones, cytokines, or other molecules; changes in the equilibrium state (i.e. half-life) or one or more RNAs, proteins, lipids, hormones, cytokines, or other molecules; changes in the localization of one or more RNAs, proteins, lipids, hormones, cytokines, or other molecules; changes in the bioactivity or specific activity of one or more RNAs, proteins, lipids, hormones, cytokines, receptors, or other molecules; changes in the secretion of ions, cytokines, hormones, growth factors, or other molecules; alterations in cellular membrane potentials, polarization, integrity or transport; changes in infectivity, susceptability, latency, adhesion, and uptake of viruses and bacterial pathogens; etc. By "capable of altering the phenotype" herein is meant that the bioactive peptide can change the phenotype of the cell in some detectable and/or measurable way.

The altered phenotype may be detected in a wide variety of ways, as is described more fully below, and will generally depend and correspond to the phenotype that is being changed. Generally, the changed phenotype is detected using, for example: microscopic analysis of cell morphology; standard cell viability assays, including both increased cell death and increased cell viability, for example, cells that are now resistant to cell death via virus, bacteria, or bacterial or synthetic toxins; standard labeling assays such as fluorometric indicator assays for the presence or level of a particular cell or molecule, including FACS or other dye staining techniques; biochemical detection of the expression of target compounds after killing the cells; etc. In some cases, as is more fully described herein, the altered phenotype is detected in the cell in which the fusion nucleic acid was introduced; in other embodiments, the altered phenotype is detected in a second cell which is responding to some molecular signal from the first cell.

An altered phenotype of a cell indicates the presence of a bioactive peptide, acting preferably in a transdominant way. By "transdominant" herein is meant that the bioactive peptide indirectly causes the altered phenotype by acting on a second molecule, which leads to an altered phenotype. That is, a transdominant expression product has an effect that is not in cis, i.e., a trans event as defined in genetic terms or biochemical terms. A transdominant effect is a distinguishable effect by a molecular entity (i.e., the encoded peptide or RNA) upon some separate and distinguishable target; that is, not an effect upon the encoded entity itself. As such, transdominant effects include many well-known effects by pharmacologic agents upon target molecules or pathways in cells or physiologic systems; for instance, the β-lactam antibiotics have a transdominant effect upon peptidoglycan synthesis in bacterial cells by binding to penicillin binding proteins and disrupting their functions. An exemplary transdominant effect by a peptide is the ability to inhibit NF-κB signaling by binding to IκB-α at a region critical for its function, such that in the presence of sufficient amounts of the peptide (or molecular entity), the signaling pathways that normally lead to the activation of NF-κB through phosphorylation and/or degradation of IκB-α are inhibited from acting at IκB-α because of the binding of the peptide or molecular entity. In another instance, signaling pathways that are normally activated to secrete IgE are inhibited in the presence of peptide. Or, signaling pathways in adipose tissue cells, normally quiescent, are activated to metabolize fat. Or, in the presence of a peptide, intracellular mechanisms for the replication of certain viruses, such as HIV-I, or Herpes viridae family members, or Respiratory Syncytia Virus, for example, are inhibited.

A transdominant effect upon a protein or molecular pathway is clearly distinguishable from randomization, change, or mutation of a sequence within a protein or molecule of known or unknown function to enhance or diminish a biochemical ability that protein or molecule already manifests. For instance, a protein that enzymatically cleaves β-lactam antibiotics, a β-lactamase, could be enhanced or diminished in its activity by mutating sequences internal to its structure that enhance or diminish the ability of this enzyme to act upon and cleave β-lactam antibiotics. This would be called a cis mutation to the protein. The effect of this protein upon β-lactam antibiotics is an activity the protein already manifests, to a distinguishable degree. Similarly, a mutation in the leader sequence that enhanced the export of this protein to the extracellular spaces wherein it might encounter β-lactam molecules more readily, or a mutation within the sequence that enhance the stability of the protein, would be termed cis mutations in the protein. For comparison, a transdominant effector of this protein would include an agent, independent of the β-lactamase, that bound to the β-lactamase in such a way that it enhanced or diminished the function of the β-lactamase by virtue of its binding to β-lactamase.

In a preferred embodiment, once a cell with an altered phenotype is detected, the presence of the fusion protein is verified, to ensure that the peptide was expressed and thus that the altered phenotype can be due to the presence of the peptide. As will be appreciated by those in the art, this verification of the presence of the peptide can be done either before, during or after the screening for an altered phenotype. This can be done in a variety of ways, although preferred methods utilize FACS techniques.

Once the presence of the fusion protein is verified, the cell with the altered phenotype is generally isolated from the plurality which do not have altered phenotypes. This may be done in any number of ways, as is known in the art, and will in some instances depend on the assay or screen. Suitable isolation techniques include, but are not limited to, FACS, lysis selection using complement, cell cloning, scanning by Fluorimager, expression of a "survival" protein, induced expression of a cell surface protein or other molecule that can be rendered fluorescent or taggable for physical isolation; expression of an enzyme that changes a non-fluorescent molecule to a fluoroscent one; overgrowth against a background of no or slow growth; death of cells and isolation of DNA or other cell vitality indicator dyes, etc.

In a preferred embodiment, the fusion nucleic acid and/or the bioactive peptide (i.e. the fusion protein) is isolated from the positive cell. This may be done in a number of ways. In a preferred embodiment, primers complementary to DNA regions common to the retroviral constructs, or to specific components of the library such as a rescue sequence, defined above, are used to "rescue" the unique random sequence. Alternatively, the fusion protein is isolated using a rescue sequence. Thus, for example, rescue sequences comprising epitope tags or purification sequences may be used to pull out the fusion protein using immunoprecipitation or affinity columns. In some instances, as is outlined below, this may also pull out the primary target molecule, if there is a sufficiently strong binding interaction between the bioactive peptide and the target molecule. Alternatively, the peptide may be detected using mass spectroscopy.

Once rescued, the sequence of the bioactive peptide and/or fusion nucleic acid is determined. This information can then be used in a number of ways.

In a preferred embodiment, the bioactive peptide is resynthesized and reintroduced into the target cells, to verify the effect. This may be done using retroviruses, or alternatively using fusions to the HIV-1 Tat protein, and analogs and related proteins, which allows very high uptake into target cells. See for example, Fawell et al., PNAS USA 91:664 (1994); Frankel et al., Cell 55:1189 (1988); Savion et al., J. Biol. Chem. 256:1149 (1981); Derossi et al., J. Biol. Chem. 269:10444 (1994); and Baldin et al., EMBO J. 9:1511 (1990), all of which are incorporated by reference.

In a preferred embodiment, the sequence of a bioactive peptide is used to generate more candidate peptides. For example, the sequence of the bioactive peptide may be the basis of a second round of (biased) randomization, to develop bioactive peptides with increased or altered activities.
Alternatively, the second round of randomization may change the affinity of the bioactive peptide. Furthermore, it may be desirable to put the identified random region of the bioactive peptide into other presentation structures, or to alter the sequence of the constant region of the presentation structure to alter the contormation/shape of the bioactive peptide. It may also be desirable to "walk" around a potential binding site, in a manner similar to the mutagenesis of a binding pocket, by keeping one end of the ligand region constant and randomizing the other end to shift the binding of the peptide around.

In a preferred embodiment, either the bioactive peptide or the bioactive nucleic acid encoding it is used to identify target molecules, i.e. the molecules with which the bioactive peptide interacts. As will be appreciated by those in the art, there may be primary target molecules, to which the bioactive peptide binds or acts upon directly, and there may be secondary target molecules, which are part of the signalling pathway affected by the bioactive peptide; these might be termed "validated targets".

In a preferred embodiment, the bioactive peptide is used to pull out target molecules. For example, as outlined herein, if the target molecules are proteins, the use of epitope tags or purification sequences can allow the purification of primary target molecules via biochemical means (co-immunoprecipitation, affinity columns, etc.). Alternatively, the peptide, when expressed in bacteria and purified, can be used as a probe against a bacterial cDNA expression library made from mRNA of the target cell type. Or, peptides can be used as "bait" in either yeast or mammalian two or three hybrid systems. Such interaction cloning approaches have been very useful to isolate DNA-binding proteins and other interacting protein components. The peptide(s) can be combined with other pharmacologic activators to study the epistatic relationships of signal transduction pathways in question. It is also possible to synthetically prepare labeled peptide and use it to screen a cDNA library expressed in bacteriophage for those cDNAs which bind the peptide. Furthermore, it is also possible that one could use cDNA cloning via retroviral libraries to "complement" the effect induced by the peptide. In such a strategy, the peptide would be required to be stochiometrically titrating away some important factor for a specific signaling pathway. If this molecule or activity is replenished by over-expression of a cDNA from within a cDNA library, then one can clone the target. Similarly, cDNAs cloned by any of the above yeast or bacteriophage systems can be reintroduced to mammalian cells in this manner to confirm that they act to complement function in the system the peptide acts upon.

Once primary target molecules have been identified, secondary target molecules may be identified in the same manner, using the primary target as the "bait". In this manner, signalling pathways may be elucidated. Similarly, bioactive peptides specific for secondary target molecules may also be discovered, to allow a number of bioactive peptides to act on a single pathway, for example for combination therapies.

The screening methods of the present invention may be useful to screen a large number of cell types under a wide variety of conditions. Generally, the host cells are cells that are involved in disease states and they are tested discreened under conditions that normally result in undesirable consequences on the cells. When a suitable bioactive peptide is found, the undesirable effect may be reduced or eliminated. Alternatively, normally desirable consequences may be reduced or eliminated, with an eye towards elucidating the cellular mechanisms associated with the disease state or signalling pathway.

In this way, fusion polypeptides comprising rGFP proteins and random peptides are made for screening of the random peptides for bioactivity.

Alternatively, the present invention provides additional fusion constructs incorporating rGFP. However, in this embodiment, the rGFP protein is fused, in a number of ways as are described herein, to a gene or regulatory element of interest.

In a preferred embodiment, the rGFP can be used to evaluate, test and screen promoters. Thus, in this embodiment, the invention provides compositions comprising a promoter of interest and a gene encoding a rGFP. Preferably the promoter is not the native rGFP promoter.

In a preferred embodiment, the invention relates to methods that rely on rGFP genes fused to IgE promoters, such as the IL-4 inducible ε promoter that starts a cascade that ultimately results in IgE production, as is generally described in U.S.S.N. 09/076,624, hereby incorporated by reference in its entirety. Using novel reporter constructs, screening for modulators of this promoter system may be done, which can be used to screen for upstream modulators of IgE production, to prevent the production of IgE and thus reduce or eliminate an allergic response. For example, an early step in the Ig switch is the production of sterile ε transcripts in response to IL-4. It is also appreciated that blockage of the production of membrane bound IgE may induce programmed cell death (PCD). By interfering at this step, highly efficient, rapid and prolonged inhibition of the allergic response may occur. In addition, these techniques allow individual cell assessment and thus are useful for high-throughput screening strategies, for example those that utilize fluorescence activated cell sorting (FACS) techniques, and thus allow screening of large numbers of compounds for their effects on IgE production.

Thus in a preferred embodiment the invention provides a number of different constructs that allow for screening for antagonists and agonists of these promoters.

In a preferred embodiment, the invention provides methods of screening for bioactive agents capable of modulating, particularly inhibiting, an IL-4 inducible ε promoter. By "an IL-4 inducible promoter" herein is meant a nucleic acid promoter that is induced by IL-4, putatively by binding an unknown IL-4 induced DNA binding protein that results in induction of the promoter, that is, the introduction of IL-4 causes the pronounced activation of a particular DNA binding protein that then binds to the IL-4 inducible promoter segment and induces transcription. The sequence of the human IL-4 inducible promoter is shown in Figure 1 of U.S.S.N. 09/076,624. hereby expressly incorporated by reference in its entirety, and as will be appreciated by those in the art, derivatives or mutant promoters are included within this definition. Particularly included within the definition of an IL-4 inducible promoter are fragments or deletions of the sequence shown in Figure 1 of U.S.S.N. 09/076,624. As is known in the art, the IL-4 inducible promoter is also inducible by IL-13. By "modulating an IL-4 inducible promoter" herein is meant either an increase or a decrease (inhibition) of promoter activity, for example as measured by the presence or quantification of transcripts or of translation products. By "inhibiting an IL-4 inducible promoter herein is meant a decrease in promoter activity, with changes of at least about 50% being preferred, and at least about 90% being particularly preferred.

The methods comprise combining a candidate bioactive agent and a cell or a population of cells comprising a fusion nucleic acid. The cell or cells comprise a fusion nucleic acid. In a preferred embodiment, the fusion nucleic acid comprises an IL-4 inducible ε promoter and at least a p- or rGFP gene. The IL-4 inducible ε promoter is as described herein, or derivatives thereof, and may be either an endogeneous or exogeneous IL-4 inducible ε promoter, as is more fully described below.

In a preferred embodiment, constructs comprising a promoter and two reporter genes can be made. In this embodiment, the first reporter gene is a rGFP gene. The second reporter gene is a death gene that provides a nucleic acid that encodes a protein that causes the cells to die. Death genes fall into two basic categories: death genes that encode death proteins that require a death ligand to kill the cells, and death genes that encode death proteins that kill cells as a result of high expression within the cell, and do not require the addition of any death ligand. It is preferable that cell death requires a two-step process: the expression of the death gene and induction of the death phenotype with a signal or ligand, such that the cells may be grown up expressing the death gene, and then induced to die. A number of death genes/ligand pairs are known, including, but not limited to, the Fas receptor and Fas ligand (Bodmer, et al., "Characterization of Fas." *J Biol Chem* 272(30):18827-18833 (Jul 25, 1997); muFAS, Gonzalez-Cuadrado, et al., "Agonistic anti-Fas Antibodies Induce Glomerular Cell Apoptosis in Mice In Vivo." *Kidney Int* 51(6):1739-1746 (Jun 1997); Muruva, et al., *Hum Gene Ther,* 8(8):955 (May 1997)). (or anti-Fas receptor antibodies); p450 and cyclophosphamide (Chen, et al., "Potentiation of Cytochrome P450/Cyclophosphamide-Based Cancer Gene Therapy By Coexpression of the P450 Reductase Gene," *Cancer Res* 57(21):4830-4837 (Nov 1 1997)); thymidine kinase and gangcylovir (Stone, R., "Molecular 'Surgery' For Brain Tumors." 256(5063):1513 (June 12, 1992)), tumor necrosis factor (TNF) receptor and TNF. Alternatively, the death gene need not require a ligand, and death results from high expression of the gene; for example, the overexpression of a number of programmed cell death (PCD) proteins are known to cause cell death, including but not limited to, caspases, bax, TRADD, FADD, SCK, MEK, etc.

In addition to the IL-4 inducible ε promoter, other promoters of interest can be used. The promoter of interest can be either a constitutive promoter or an inducible promoter, such as the IL-4 inducible ε promoter. As will be appreciated by those in the art, any number of possible promoters could be used.

Suitable promoters of interest include, but are not limited to, inducible promoters such as IL-4 ε promoter, promoters that are induced by cytokines or growth factors such as the interferon responsive factors 1 to 4. NFkB (Fiering, et al., "Single Cell Assay of a Transcription Factor Reveals a Threshold in Transcription Activated By Signals Emanating From the T-Cell Antigen Receptor," *Genes Dev* 4(10):1823-1834 (Oct 1990)), promoters activated by heavy metals, heat shock promoters, stress promoters, etc. When inducible promoters are used in this embodiment, suitable cell types are those that can be induced by the appropriate inducer, as will be appreciated by those in the art. Constitutive promoters are also of use, particularly tissue specific promoters, including, but not limited to, CNS, PNS, brain, kidney, skin, bone, lung, heart, liver, bladder, ovary, testes, colon, etc. specific promoters.

In a preferred embodiment, the promoter of interest is a constitutive promoter, and it is hooked to a death gene that requires the presence of a ligand, such as Fas or TNF. Thus, the cells can be grown up and the presence of the death gene verified due to the constitutive promoter. This is done by hooking the death gene up to a rGFP gene, using either an IRES or a protease cleavage site as is outlined below; thus, the presence of the rGFP gene means the death gene is also present. Verification of the presence of the death gene is preferred to keep the levels of false positives low; that is, cells that survive the screen should be due to the presence of an inhibitor of the promoter rather than a lack of the death gene.

Once the cells have been enriched for those containing the death gene, the candidate agents can be added (and their presence verified as well), followed by induction in the presence of IL-4, and finally by addition of the death ligand. Thus, the cell population is enriched for those cells that have an agent that inhibits the promoter and thus does not produce the death protein, i.e. those that survive.

When death genes that require ligands are used, i.e. for "two step" processes, preferred embodiments utilize chimeric death genes, i.e. chimeric death receptor genes. These chimeric death receptors comprise the extracellular domain of a ligand-activated multimerizing receptor and the endogeneous cytosolic domain of a death receptor gene, such as Fas or TNF. This is done to avoid endogeneous activation of the death gene. The mechanism of Fas-induced cell death involves the introduction of the Fas ligand, which can bind two monomeric Fas receptors, causing the multimerization of the receptor, which activates the receptor and leads to secondary signalling resulting in caspase activation and PCD. However, as will be appreciated by those in the art, it is possible to substitute the extracellular portion of the death receptor with the extracellular portion of another ligand-activated multimerizing receptor, such that a completely different signal activates the cell to die. There are a number of known ligand-activated dimerizing receptors, including, but not limited to, the CD8 receptor, erythropoeitin receptor, thrombopoeitin receptor, growth hormone receptor, Fas receptor, platelet derived growth hormone receptor, epidermal growth factor receptor, leptin receptor, and a variety of interleukin receptors (including, but not limited to, IL-1, IL-2, IL-3, IL-4, IL-5, IL-6, IL-7, IL-8, IL-9, IL-11, IL-12, IL-13, IL-15, and IL-17; although the use of the IL-4 and IL-13 receptors are not preferred, since these can be used to induce the promoter and thus does not provide a "two step" death process), low-density lipoprotein receptor, prolactin receptor, and transferrin receptor.

In a preferred embodiment, chimeric Fas receptor genes are made. The exact combination will depend on the cell type used and the receptors normally produced by these cells. For example, when using human cells or cell lines, a non-human extracellular domain and a human cytosolic domain are preferred, to prevent endogeneous induction of the death gene. For example, a preferred embodiment utilizes human cells, a murine extracellular Fas receptor domain and a human cytosolic domain, such that the endogeneous human Fas ligand will not activate the murine domain. Alternatively, human extracellular domains may be used when the cells used do not endogeneously produce the ligand; for example, the human EPO extracellular domain may be used when the cells do not endogeneously produce EPO. (Kawaguchi, et al., *Cancer Lett.,* 116(1):53 (1997); Takebayashi, et al., *Cancer Res.,* 56(18):4164 (1996); Rudert, et al., *Biochem Biophys Res Commun.,* 204(3):1102 (1194); Rudert, et al., *DNA Cell Biol.,* 16(2):197 (1997); Takahasi, et al., *J Biol Chem.* 271(29):17555 (1996); Adam, et al., *J Biol Chem.,* 268(26):19882 (1993); Mares, et al., *Growth Factors,* 6(2):93 (1992); Seedorf, et al., *J Biol Chem.,* 266(19):12424 (1991); Heidaran, et al., *J Biol Chem.,* 265(31):18741 (1990); Okuda, et al., *J Clin Invest.* 100(7):1708 (1997); Allgood, et al., *CurGFP Opin Biotechnol.,* 8(4):474 (1997); Anders, et al., *J Biol Chem.,* 271(36):21758 (1996); Krishnan, et al., *Oncogene,* 13(1):125 (1996); Declercq, et al., *Cytokine,* 7(7):701 (1995); Bazzoni, et al., *Proc Natl Acad Sci U S.,* 92(12):5380 (1995); Ohashi, et al., *Proc Natl Acad Sci U S A ,* 91(1):158 (1994); Desai, et al., *Cell,* 73(3):541 (1993); and Amara, et al., *Proc Natl Acad Sci U S A,* 94(20):10618 (1997)).

In addition to the extracellular domain and the cytosolic domain, these receptors have a transmembrane domain. As will be appreciated by those in the art, for chimeric death receptor genes, the transmembrane domain from any of the receptors can be used, although in general, it is preferred to use the transmembrane domain associated with the chosen cytosolic domain, to preserve the interaction of the transmembrane domain with other endogeneous signalling proteins.

Thus, preferred embodiments provide fusion nucleic acids that utilize the IL-4 inducible ε promoter linked to a p- or rGFP gene and a death gene, particularly a chimeric death receptor gene, that requires a death ligand for cell killing, particularly with an IRES in between the reporter genes..

Alternatively, inducible promoters can be linked to "one step" death genes, i.e. death genes that upon a certain threshold expression, will kill a cell without requiring a ligand or secondary signal. In this embodiment, the inducible promoter is preferably "leaky", such that some small amount of death gene and a required secondary reporter gene such as a survival gene or a detection gene can be expressed. The cells that contain the death gene can then be selected on this basis, to avoid false positives. Once the presence of the construct is verified, candidate agents are added (and their presence preferably verified, using a detection or selection gene as well), and the promoter is induced. The population is then enriched for those cells that contain agents that inhibit the promoter, i.e. that will survive. In this embodiment, a rGFP gene is used, particularly when inducible death genes are used. The use of a rGFP gene allows cells to be sorted to give a population enriched for those containing the construct. As outlined above,a preferred embodiment uses "leaky" inducible promoters; that is, the cells are selected such that the IL-4 inducible promoter, even in the absence of IL-4 or IL-13, produces some rGFP and death gene (for example, the Fas receptor constructs). In this embodiment, suitably "leaky" promoters are chosen such that some rGFP is expressed (preferably enough to select the cells expressing the construct from those that are not), but not enough death gene is produced to cause death. While preferred embodiments utilize death genes requiring the addition of a death ligand, it is well known that high levels of some death genes, even-in the absence of death ligand, can cause death. Thus, for example, high levels of Fas receptor expression can cause multimerization, and thus activation, even in the absence of the Fas ligand.

In a preferred embodiment, when two reporter genes are used, they are fused together in such a way as to only require a single promoter, and thus some way of functionally separating the two genes is preferred. This can be done on the RNA level or the protein level. Preferred embodiments utilize either IRES sites (which allows the translation of two different genes on a single transcript (Kim, et al., "Construction of a Bifunctional mRNA in the Mouse By Using the Internal Ribosomal Entry Site of the Encephalomycarditis Virus," *Molecular and Cellular Biology* 12(8):3636-3643 (Aug 1992) and McBratney, et al., "The Sequence Context of the Initiation Codon in the Encephalomycarditis Virus Leader Modulates Efficiency of Internal Translation Initiation," *Current Opinion in Cell Biology* 5:961-965 (1993)), or a protease cleavage site (which cleaves a protein translation product into two proteins). Preferred protease cleavage sites include, but are not limited to, the 2a site (Ryan et al., J. Gen. Virol. 72:2727 (1991); Ryan et al., EMBO J. 13:928 (1994); Donnelly et al., J. Gen. Virol. 78:13 (1997); Hellen et al., *Biochem,* 28(26):9881 (1989); and Mattion et al., J. Virol. 70:8124 (1996), all of which are expressly incorporated by reference), prosequences of retroviral proteases including human immunodeficiency virus protease and sequences recognized and cleaved by trypsin (EP 578472. Takasuga et al., J. Biochem. 112(5)652 (1992)) factor Xa (Gardella et al., J. Biol. Chem. 265(26):15854 (1990), WO 9006370), collagenase (J03280893, Tajima et al., J. Ferment. Bioeng. 72(5):362 (1991), WO 9006370), clostripain (EP 578472), subtilisin (including mutant H64A subtilisin, Forsberg et al., J. Protein Chem. 10(5):517 (1991), chymosin, yeast KEX2 protease (Bourbonnais et al., J. Bio. Chem. 263(30):15342 (1988), thrombin (Forsberg et al., supra; Abath et al., BioTechniques 10(2):178 (1991)), *Staphylococcus aureus* V8 protease or similar endoproteinase-Glu-C to cleave after Glu residues (EP 578472, lshizaki et al., Appl. Microbiol. Biotechnol. 36(4):483 (1992)), cleavage by Nla proteainase of tobacco etch virus (Parks et al., Anal. Biochem. 216(2):413 (1994)), endoproteinase-Lys-C (U.S. Patent No. 4,414,332) and endoproteinase-Asp-N. Neisseria type 2 IgA protease (Pohlner et al., Sio/Technology 10(7):799-804 (1992)), soluble yeast endoproteinase yscF (EP 467839), chymotrypsin (Altman et al., Protein Eng. 4(5):593 (1991)), enteropeptidase (WO 9006370), lysostaphin, a polyglycine specific endoproteinase (EP 316748), and the like. See e.g. Marston, F.A.O. (1986) Biol. Chem. J. **240**, 1-12.

Thus, in preferred embodiment, fusion constructs comprising a gene of interest, an IRES site and an rGFP gene are provided.

In addition to the promoter of interest, such as an IL-4 inducible ε promoter and rGFP gene, the fusion nucleic acids may comprise additional components, including, but not limited to, other reporter genes, protein cleavage sites, internal ribosome entry (IRES) sites, AP-1 sites, and other components as will be appreciated by those in the art.

In a preferred embodiment, foreign constructs comprising the IL-4 inducible ε promoter and the rGFP gene are made. By "foreign" herein is meant that the fusion nucleic acids originates outside of the cells. That is, a recombinant nucleic add is made that contains an exogeneous IL-4 inducible e promoter and an rGFP gene. Thus, in some circumstances, the cells will contain both exogeneous and endogeneous IL-4 inducible e promoters. By "recombinant nucleic acid" herein is meant nucleic acid, originally formed *in vitro,* in general, by the manipulation of nucleic acid by endonucleases, in a form not normally found in nature. Thus an isolated nucleic acid, in a linear form, a nucleic acid containing components not normally joined, such as an non-rGFP promoter and an rGFP gene, or an expression vector formed *in vitro* by ligating DNA molecules that are not normally joined, are all considered recombinant for the purposes of this invention. It is understood that once a recombinant nucleic acid is made and reintroduced into a host cell or organism, it will replicate non-recombinantly, i.e. using the in vivo cellular machinery of the host cell rather than *in vitro* manipulations; however, such nucleic adds, once produced recombinantly, although subsequently replicated non-recombinantly, are still considered recombinant for the purposes of the invention.

For the IL-4 inducible ε promoter systems, any cells that express an IL-4 receptor that transduces the IL-4 signal to the nucleus and alters transcription can be used. Suitable cells include, but are not limited to, human cells and cell lines that show IL-4/13 inducible production of germline ε transcripts, including, but not limited to, DND39 (see Watanabe, supra), MC-116, (Kumar, et al., "Human BCGF-12kD Functions as an Autocrine Growth Factor in Transformed B Cells," *Eur Cytokine Netw* 1(2):109 (1990)), CA-46 (Wang, et al., "UCN-01: A Potent Abrogator of G2 Checkpoint Function in Cancer Cells with Dirupted p53," *J Natl Cancer Inst* 88:956 (1996)).

As for all the embodiments outlined herein, the recombinant nucleic acid (e.g. the fusion nucleic acids) may be introduced to a cell in a variety of ways, as will be appreciated by those in the art, including, but not limited to, CaPO₄ precipitation, liposome fusion, lipofectin®, electroporation, viral infection, etc. The constructs may preferably stably integrate into the genome of the host cell (for example, with retroviral introduction, outlined below), or may exist either transiently or stably in the cytoplasm (i.e. through the use of traditional plasmids, utilizing standard regulatory sequences, selection markers, etc.).

In a preferred embodiment, the exogeneous constructs, which may be in the form of an expression vector, are added as retroviral constructs, using techniques generally described in PCT US97/01019 and PCT US97/01048, both of which are expressly incorporated by reference in their entirety.

In a preferred embodiment, the fusion construct comprises an endogeneous promoter (such as an IL-4 inducible ε promoter) and an exogeneous rGFP gene; "endogeneous" in this context means originating within the cell. That is, gene "knock-in" constructions are made, whereby an exogeneous rGFP gene as outlined herein is added, via homologous recombination, to the genome, such that the reporter gene is under the control of the endogeneous promoter. This may be desirable to allow for the exploration and modulation of the full range of endogeneous regulation, i.e. regulatory elements (particularly those flanking the promoter) other than just the promoter fragment.

Homologous recombination may proceed in several ways. In one embodiment, traditional homologous recombination is done, with molecular biological techniques such as PCR being done to find the correct insertions. For example, gene "knock-ins" may be done as is known in the art, for example see Westphal et al., Current Biology 7:R530-R533 (1997), and references cited therein, all of which are expressly incorporated by reference. The use of recA mediated systems may also be done, see PCT US93/03868, hereby expressly incorporated by reference.

Alternatively, and preferably, the selection of the "knock ins" are done by FACS on the basis of the incorporation of the rGFP gene. Thus, in a preferred embodiment, a first homologous recombination event is done to put an rGFP gene, into at least one allele of the cell genome. When the promoter is the IL-4 inducible promoter, preferably, this is a cell type that exhibits IL-4 inducible production of at least germline ε transcripts, so that the cells may be tested by IL-4 production for reporter gene expression. Suitable cells include, but are not limited to, human cells and cell lines that show IL-4/13 inducible production of germline ε transcripts, including, but not limited to, DND39 (see Watanabe, supra), MC-116, (Kumar, et al., "Human BCGF-12kD Functions as an autocrine Growth Factor in Transformed B Cells," *Eur Cytokine Netw* 1(2):109 (1990)), CA-46 (Wang, et al., "UCN-01:A Potent Abrogator of G2 Checkpoint Function in Cancer Cells with Dirupted p53," *J Natl Cancer Inst* 88:956 (1996)). As is noted herein, the ability of MC-116 and CA-46 cells to produce germline ε transcripts upon IL-4/13 induction was not known prior to the present invention. Thus, preferred embodiments provide MC-116 and/or CA-46 cells comprising recombinant nucleic acid reporter constructs are outlined herein.

As will be appreciated by those in the art and outlined herein, any number of suitable cell types can be used in the present invention.

In a preferred embodiment, once a first endogeneous promoter has been combined with an exogeneous reporter construct, a second homologous recombination event may be done, preferably using a second reporter gene different from the first, to target the other allele of the cell genome, and tested as above.

Generally, IL-4 induction of the rGFP genes will indicate the correct placement of the genes, which can be confirmed via sequencing such as PCR sequencing or Southern blot hybridization. In addition, preferred embodiments utilize prescreening steps to remove 'leaky' cells, i.e. those showing constitutive expression of the p- or rGFP gene.

Thus, in a preferred embodiment, the invention provides cell lines that contain fusion nucleic acids comprising IL-4 inducible ε promoter operably connected to an rGFP gene. Once made, the cell lines comprising these reporter constructs are used to screen candidate bioactive agents for the ability to modulate the production of IgE, as is outlined below.

The term "candidate bioactive agent" or "exogeneous compound" as used herein describes any molecule, *e.g*., protein, oligopeptide, small organic molecule, polysaccharide, polynucleotide.
Generally a plurality of assay mixtures are run in parallel with different agent concentrations to obtain a differential response to the various concentrations. Typically, one of these concentrations serves as a negative control, *i.e.,* at zero concentration or below the level of detection.

Candidate agents encompass numerous chemical classes, though typically they are organic molecules, preferably small organic compounds having a molecular weight of more than 100 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof. Particularly preferred are peptides.

Candidate agents are obtained from a wide variety of sources including libraries of synthetic or natural compounds. For example, numerous means are available for random and directed synthesis of a wide variety of organic compounds and biomolecules, including expression of randomized oligonucleotides. Alternatively, libraries of natural compounds in the form of bacterial, fungal, plant and animal extracts are available or readily produced. Additionally, natural or synthetically produced libraries and compounds are readily modified through conventional chemical, physical and biochemical means. Known pharmacological agents may be subjected to directed or random chemical modifications, such as acylation, alkylation, esterification, amidification to produce structural analogs.

In a preferred embodiment, the candidate bioactive agents are proteins. By "protein" herein is meant at least two covalently attached amino acids, which includes proteins, polypeptides, oligopeptides and peptides. The protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and noreleucine are considered amino acids for the purposes of the invention. "Amino acid" also includes imino acid residues such as proline and hydroxyproline. The side chains may be in either the (R) or the (S) configuration. In the preferred embodiment, the amino acids are in the (S) or L-configuration. If non-naturally occurring side chains are used, non-amino acid substituents may be used, for example to prevent or retard *in vivo* degradations.

In a preferred embodiment, the candidate bioactive agents are naturally occuring proteins or fragments of naturally occuring proteins. Thus, for example, cellular extracts containing proteins, or random or directed digests of proteinaceous cellular extracts, may be used. In this way libraries of procaryotic and eucaryotic proteins may be made for screening in the systems described herein. Particularly preferred in this embodiment are libraries of bacterial, fungal, viral, and mammalian proteins, with the latter being preferred, and human proteins being especially preferred.

In a preferred embodiment, the candidate bioactive agents are peptides of from about 5 to about 30 amino acids, with from about 5 to about 20 amino acids being preferred, and from about 7 to about 15 being particularly preferred. The peptides may be digests of naturally occuring proteins as is outlined above, random peptides, or "biased" random peptides. By "randomized" or grammatical equivalents herein is meant that each nucleic acid and peptide consists of essentially random nucleotides and amino acids, respectively. Since generally these random peptides (or nucleic acids, discussed below) are chemically synthesized, they may incorporate any nucleotide or amino acid at any position. The synthetic process can be designed to generate randomized proteins or nucleic acids, to allow the formation of all or most of the possible combinations over the length of the sequence, thus forming a library of randomized candidate bioactive proteinaceous agents.

In one embodiment, the library is fully randomized, with no sequence preferences or constants at any position. In a preferred embodiment, the library is biased. That is, some positions within the sequence are either held constant, or are selected from a limited number of possibilities. For example, in a preferred embodiment, the nucleotides or amino acid residues are randomized within a defined class, for example, of hydrophobic amino acids, hydrophilic residues, sterically biased (either small or large) residues, towards the creation of cysteines, for cross-linking, prolines for SH-3 domains, serines, threonines, tyrosines or histidines for phosphorylation sites, etc., or to purines, etc.

In a preferred embodiment, the candidate bioactive agents are nucleic acids. By "nucleic acid" or "oligonucleotide" or grammatical equivalents herein means at least two nucleotides covalently linked together. A nucleic acid of the present invention will generally contain phosphodiester bonds, although in some cases, as outlined below, nucleic acid analogs are included that may have alternate backbones, comprising, for example, phosphoramide (Beaucage, *et al.,* Tetrahedron, **49**(10):1925 (1993) and references therein; Letsinger, J. Org. Chem., **35**:3800 (1970); Sprinzl, *et al.,* Eur. J. Biochem., **81**:579 (1977); Letsinger, *et* a/., Nucl. Acids Res., **14**:3487 (1986); Sawai, *et al*., Chem. Lett., 805 (1984), Letsinger, *et al.,* J. Am. Chem. Soc., **110:**4470 (1988); and Pauwels, *et al.,* Chemica Scripta, **26**:141 (1986)), phosphorothioate (Mag, *et al.,* Nucleic Acids Res., 19:1437 (1991); and U.S. Patent No. 5,644,048), phosphorodithioate (Briu, *et al.,* J. Am. Chem. Soc., **111:**2321 (1989)), O-methylphophoroamidite linkages (see Eckstein, Oligonucleotides and Analogues: A Practical Approach, Oxford University Press), and peptide nucleic acid backbones and linkages (see Egholm, J. Am. Chem. Soc., **114:**1895 (1992); Meier, *et al.,* Chem. Int. Ed. Engl., 31:1008 (1992); Nielsen, Nature, **365**:566 (1993); Carlsson, *et al.,* Nature, **380**:207 (1996), all of which are incorporated by reference)). Other analog nucleic acids include those with positive backbones (Denpcy, *et al.,* Proc. Natl. Acad. Sci. USA, **92**:6097 (1995)); non-ionic backbones (U.S. Patent Nos. 5,386,023; 5,637,684: 5,602,240; 5,216,141; and 4,469,863; Kiedrowshi, *et al.,* Angew. Chem. Intl. Ed. English, 30:423 (1991); Letsinger, *et al.,* J. Am. Chem. Soc., **110**:4470 (1988); Letsinger, *et al.,* Nucleoside & Nucleotide, 13:1597 (1994); Chapters 2 and 3, ASC Symposium Series 580, "Carbohydrate Modifications in Antisense Research", Ed. Y.S. Sanghui and P. Dan Cook; Mesmaeker, et *al.,* Bioorganic & Medicinal Chem. Lett., **4**:395 (1994); Jeffs, *et al.,* J. Biomolecular NMR, **34**:17 (1994); Tetrahedron Lett., **37**:743 (1996)) and non-ribose backbones, including those described in U.S. Patent Nos. 5,235,033 and 5,034,506, and Chapters 6 and 7, ASC Symposium Series 580, "Carbohydrate Modifications in Antisense Research", Ed. Y.S. Sanghui and P. Dan Cook. Nucleic acids containing one or more carbocyclic sugars are also included within the definition of nucleic acids (see Jenkins, *et al.,* Chem. Soc. Rev., (1995) pp. 169-176). Several nucleic acid analogs are described in Rawls, C & E News, June 2, 1997, page 35. All of these references are hereby expressly incorporated by reference. These modifications of the ribose-phosphate backbone may be done to facilitate the addition of additional moieties such as labels, or to increase the stability and half-life of such molecules in physiological environments. In addition, mixtures of naturally occurring nucleic acids and analogs can be made. Alternatively, mixtures of different nucleic acid analogs, and mixtures of naturally occuring nucleic acids and analogs may be made. The nucleic acids may be single stranded or double stranded, as specified, or contain portions of both double stranded or single stranded sequence. The nucleic acid may be DNA, both genomic and cDNA, RNA or a hybrid, where the nucleic acid contains any combination of deoxyribo- and ribo-nucleotides, and any combination of bases, including uracil, adenine, thymine, cytosine, guanine, inosine, xathanine hypoxathanine, isocytosine, isoguanine, etc.

As described above generally for proteins, nucleic acid candidate bioactive agents may be naturally occuring nucleic acids, random nucleic acids, or "biased" random nucleic acids. For example, digests of procaryotic or eucaryotic genomes may be used as is outlined above for proteins.

In a preferred embodiment, the candidate bioactive agents are organic chemical moieties, a wide variety of which are available in the literature.

In a preferred embodiment, a library of different candidate bioactive agents are used. Preferably, the library should provide a sufficiently structurally diverse population of randomized agents to effect a probabilistically sufficient range of diversity to allow binding to a particular target. Accordingly, an interaction library should be large enough so that at least one of its members will have a structure that gives it affinity for the target. Although it is difficult to gauge the required absolute size of an interaction library, nature provides a hint with the immune response: a diversity of 10⁷-10⁸ different antibodies provides at least one combination with sufficient affinity to interact with most potential antigens faced by an organism. Published *in vitro* selection techniques have also shown that a library size of 10⁷ to 10⁸ is sufficient to find structures with affinity for the target. A library of all combinations of a peptide 7 to 20 amino acids in length, such as generally proposed herein, has the potential to code for 20⁷ (10⁹) to 20²⁰. Thus, with libraries of 10⁷ to 10⁸ different molecules the present methods allow a "working" subset of a theoretically complete interaction library for 7 amino acids, and a subset of shapes for the 20²⁰ library. Thus, in a preferred embodiment, at least 10⁶, preferably at least 10⁷, more preferably at least 10⁸ and most preferably at least 10⁹ different sequences are simultaneously analyzed in the subject methods. Preferred methods maximize library size and diversity.

The candidate bioactive agents are combined or added to a cell or population of cells. Suitable cell types for different embodiments are outlined above. By "population of cells" herein is meant at least two cells, with at least about 10⁵ being preferred, at least about 10⁶ being particularly preferred, and at least about 10⁷, 10⁸ and 10⁹ being especially preferred.

The candidate bioactive agent and the cells are combined. As will be appreciated by those in the art, this may accomplished in any number of ways, including adding the candidate agents to the surface of the cells, to the media containing the cells, or to a surface on which the cells are growing or in contact with; adding the agents into the cells, for example by using vectors that will introduce the agents into the cells (i.e. when the agents are nucleic acids or proteins).

In a preferred embodiment, the candidate bioactive agents are either nucleic acids or proteins (proteins in this context includes proteins, oligopeptides, and peptides) that are introduced into the host cells using retroviral vectors, as is generally outlined in PCT US97/01019 and PCT US97/01048, both of which are expressly incorporated by reference. Generally, a library of retroviral vectors is made using retroviral packaging cell lines that are helper-defective and are capable of producing all the necessary trans proteins, including gag, pol and env, and RNA molecules that have in cis the ψ packaging signal. Briefly, the library is generated in a retrovirus DNA construct backbone; standard oligonucleotide synthesis is done to generate either the candidate agent or nucleic acid encoding a protein, for example a random peptide, using techniques well known in the art. After generation of the DNA library, the library is cloned into a first primer. The first primer serves as a "cassette", which is inserted into the retroviral construct. The first primer generally contains a number of elements, including for example, the required regulatory sequences (e.g. translation, transcription, promoters, etc), fusion partners, restriction endonuclease (cloning and subcloning) sites, stop codons (preferably in all three frames), regions of complementarity for second strand priming (preferably at the end of the stop codon region as minor deletions or insertions may occur in the random region), etc.

A second primer is then added, which generally consists of some or all of the complementarity region to prime the first primer and optional necessary sequences for a second unique restriction site for subcloning. DNA polymerase is added to make double-stranded oligonucleotides. The double-stranded oligonucleotides are cleaved with the appropriate subcloning restriction endonucleases and subcloned into the target retroviral vectors, described below.

Any number of suitable retroviral vectors may be used. Generally, the retroviral vectors may include: selectable marker genes under the control of internal ribosome entry sites (IRES) that greatly facilitates the selection of cells expressing peptides at uniformly high levels; and promoters driving expression of a second gene, placed in sense or anti-sense relative to the 5' LTR. Suitable selection genes include, but are not limited to, neomycin, blastocidin, bleomycin, puromycin, and hygromycin resistance genes, as well as self-tluorescent markers such as green fluoroscent protein including rr, enzymatic markers such as IacZ, and surface proteins such as CDB, etc.

Preferred vectors include a vector based on the murine stem cell virus (MSCV) (see Hawley et al., Gene Therapy 1:136 (1994)) and a modified MFG virus (Rivere et al., Genetics 92:6733 (1995)), and pBABE. outlined in the examples.

The retroviruses may include inducible and constitutive promoters for the expression of the candidate agent (to be distinguished from the IL-4 inducible e promoter). For example, there are situations wherein it is necessary to induce peptide expression only during certain phases of the selection process. A large number of both inducible and constitutive promoters are known.

In addition, it is possible to configure a retroviral vector to allow inducible expression of retroviral inserts after integration of a single vector in target cells; importantly, the entire system is contained within the single retrovirus. Tet-inducibte retroviruses have been designed incorporating the Self-Inactivating (SIN) feature of 3' LTR enhancer/promoter retroviral deletion mutant (Hoffman et al., PNAS USA 93:5185 (1996)). Expression of this vector in cells is virtually undetectable in the presence of tetracycline or other active analogs. However, in the absence of Tet, expression is turned on to maximum within 48 hours after induction, with uniform increased expression of the whole population of cells that harbor the inducible retrovirus, indicating that expression is regulated uniformly within the infected cell population. A similar, related system uses a mutated Tet DNA-binding domain such that it bound DNA in the presence of Tet, and was removed in the absence-of Tet. Either of these systems is suitable.

In a preferred embodiment, the candidate bioactive agents are linked to a fusion partner as defined above.

In a preferred embodiment, the invention provides compositions and methods utilizing rGFP as a reporter molecule for use in cell assays. As will be appreciated by those in the art, any assay for which a reporter gene can be used can be run using rGFP.

In a preferred embodiment, the present invention provides compositions and methods utilizing rGFP and a chip device comprising integrated photodetectors at individual loci. The method may be practiced with any suitable chip device that includes an electronic circuit capable of reading the sensed signal generated by each photodetector and generating output data signals therefrom. The output data signals are indicative of the light emitted, due to the presence of rGFP, at the various loci. As will be appreciated by those in the art, any assay that evaluates binding interactions can utilize the present invention.

Thus, the present invention finds use in a variety of assays, including but not limited to, assays for protein-protein interactions, protein-nucleic acid interactions, and nucleic acid-nucleic acid interactions.

In a preferred embodiment, any cellular assay that evaluates the effects of candidate agents, preferably either nucleic acids or proteins (including peptide), can utilize the present invention. In this embodiment, the candidate agents are fused to the rGFP proteins of the present invention, generally through making fusion nucleic acids and transforming into the cells to be assayed under conditions that allow expression (if peptides are used) of the candidate agent. This allows a confirmation that the candidate agent has been expressed, as well as tracking and localization of the candidate agent, and the ability to sort cells comprising the candidate agents.

Thus, the present invention finds use in a variety of cellular assays, including but not limited to, assays for alterations in exocytosis, cell cycle regulation, apoptosis, cellular proliferation and/or differentation, etc. The cells screened can also be a variety of cell types, including, but not limited to, any cells outlined herein, including mast cells, T cells, B cells, macrophages, adipocytes, smooth muscle cells, etc.

In addition, as outlined herein, the rGFP proteins of the invention find particular use in screening assays that require a reporter protein, as outlined below.

The present invention is directed to the detection of alterations in cellular phenotypes, such as cell cycle regulation, exocytosis, small molecule toxicity, cell surface receptor expression, enzyme expression, etc. by evaluating or assaying a variety of cellular parameters, generally through the use of a fluorescence-activated cell sorter (FACS) machine. There are a number of parameters that can be measured to allow detection of alterations in a variety of cellular phenotypes as is more fully outlined below. By assaying a plurality of these parameters either sequentially or preferably simultaneously, rapid and accurate screening may be done.

In a preferred embodiment, the methods outlined herein are used to screen for modulators of cellular phenotypes. Cellular phenotypes that may be assayed include, but are not limited to, cellular apoptosis, including cell cycle regulation, exocytosis, toxicity to small molecules, the expression of any number of moieties including receptors (particularly cell surface receptors), adhesion molecules, cytokine secretion, protein-protein interactions, etc. As will be appreciated by those in the art, any number of cellular assays that rely on p- or rGFP can be developed. Thus, in a preferred embodiment, the invention provides methods of screening comprising providing cell lines comprising nucleic acids encoding an rGFP protein, adding candidate bioactive agents and detecting changes in chellular phenotype. The nucleic acid may preferably be a fusion nucleic acid, encoding a gene or regulatory element of interest operably linked to an rGFP protein.

In a preferred embodiment, the methods are used to evaluate cell cycle regulation. In this embodiment, preferred cellular parameters or assays are cell viability assays, assays to determine whether cells are arrested at a particular cell cycle stage ("cell proliferation assays"), and assays to determine at which cell stage the cells have arrested ("cell phase assays"). By assaying or measuring one or more of these parameters, it is possible to detect not only alterations in cell cycle regulation, but alterations of different steps of the cell cycle regulation pathway. This may be done to evaluate native cells, for example to quantify the aggressiveness of a tumor cell type, or to evaluate the effect of candidate drug agents that are being tested for their effect on cell cycle regulation. In this manner, rapid, accurate screening of candidate agents may be performed to identify agents that modulate cell cycle regulation.

Thus, the present methods are useful to elucidate bioactive agents that can cause a population of cells to either move out of one growth phase and into another, or arrest in a growth phase. In some embodiments, the cells are arrested in a particular growth phase, and it is desirable to either get them out of that phase or into a new phase. Alternatively, it may be desirable to force a cell to arrest in a phase, for example G1, rather than continue to move through the cell cycle. Similarly, it may be desirable in some circumstances to accelerate a non-arrested but slowly moving population of cells into either the next phase or just through the cell cycle, or to delay the onset of the next phase. For example, it may be possible to alter the activities of certain enzymes, for example kinases, phosphatases, proteases or ubiquitination enzymes, that contribute to initiating cell phase changes.

In a preferred embodiment, the methods outlined herein are done on cells that are not arrested in the G1 phase; that is, they are rapidly or uncontrollably growing and replicating, such as tumor cells. In this manner, candidate agents are evaluated to find agents that can alter the cell cycle regulation, i.e. cause the cells to arrest at cell cycle checkpoints, such as in G1 (although arresting in other phases such as S, G2 or M are also desirable). Alternatively, candidate agents are evaluated to find agents that can cause proliferation of a population of cells, i.e. that allow cells that are generally arrested in G1 to start proliferating again; for example, peripheral blood cells, terminally differentiated cells, stem cells in culture, etc.

Accordingly, in a preferred embodiment, the invention provides methods for screening for alterations in cell cycle regulation of a population of cells. "Alteration" and "modulation" (used herein interchangeably), as used herein can include both increases and decreases in the parameter or phenotype being measured. By "alteration" or "modulation" in the context of cell cycle regulation, is generally meant one of two things. In a preferred embodiment, the alteration results in a change in the cell cycle of a cell, i.e. a proliferating cell arrests in any one of the phases, or an arrested cell moves out of its arrested phase and starts the cell-cycle, as compared to another cell or in the same cell under different conditions. Alternatively, the progress of a cell through any particular phase may be altered; that is, there may be an acceleration or delay in the length of time it takes for the cells to move thorough a particular growth phase. For example, the cell may be normally undergo a G1 phase of several hours; the addition of an agent may prolong the G1 phase.

The measurements can be determined wherein all of the conditions are the same for each measurement, or under various conditions, with or without bioactive agents, or at different stages of the cell cycle process. For example, a measurement of cell cycle regulation can be determined in a cell population wherein a candidate bioactive agent is present and wherein the candidate bioactive agent is absent. In another example, the measurements of cell cycle regulation are determined wherein the condition or environment of the populations of cells differ from one another. For example, the cells may be evaluated in the presence or absence of physiological signals, for example hormones, antibodies, peptides, antigens, cytokines, growth factors, action potentials, pharmacological agents (i.e. chemotherapeutics, etc.), or other cells (i.e. cell-cell contacts). In another example, the measurements of cell cycle regulation are determined at different stages of the cell cycle process. In yet another example, the measurements of cell cycle regulation are taken wherein the conditions are the same, and the alterations are between one cell or cell population and another cell or cell population.

In a preferred embodiment, the candidate bioactive agents are peptides and are fused with rGFP proteins; fusion nucleic acids are made, transformed into the cells and expressed. The presence of a signal from the rGFP protein shows that the candidate agent is expressed. The cells can then be screened as below, to detect agents that effect cell viability, etc.

By a "population of cells" or "library of cells" or "plurality of cells" herein is meant at least two cells, with at least about 10³ being preferred, at least about 10⁶ being particularly preferred, and at least about 10⁸ to 10⁹ being especially preferred. The population or sample can contain a mixture of different cell types from either primary or secondary cultures although samples containing only a single cell type are preferred, for example, the sample can be from a cell line, particularly tumor cell lines (particularly when , as outlined below. The cells may be in any cell phase, either synchronously or not, including M, G1, S, and G2. In a preferred embodiment, cells that are replicating or proliferating are used; this may allow the use of retroviral vectors for the introduction of candidate bioactive agents. Alternatively, non-replicating cells may be used, and other vectors (such as adenovirus and lentivirus vectors) can be used. In addition, although not required, the cells are compatible with dyes and antibodies.

Preferred cell types for use in the invention will vary with the cellular phenotype to be modulated. Suitable cells include, but are not limited to, mammalian cells, including animal (rodents, including mice, rats, hamsters and gerbils), primates, and human cells, particularly including tumor cells of all types, including breast, skin, lung, cervix, colonrectal, leukemia, brain, etc. As outlined below, additional cell types may be used for screening for exocytosis.

In a preferred embodiment, the cell cycle regulation methods comprise sorting the cells in a FACS machine by assaying several different cell parameters, including, but not limited to, cell viability, cell proliferation, and cell phase.

In a preferred embodiment, cell viability is assayed, to ensure that a lack of cellular change is due to experimental conditions (i.e. the introduction of a candidate bioactive agent) not cell death. There are a variety of suitable cell viability assays which can be used, including, but not limited to, light scattering, viability dye staining, and exclusion dye staining.

In a preferred embodiment, a light scattering assay is used as the viability assay, as is well known in the art. When viewed in the FACS, cells have particular characteristics as measured by their forward and 90 degree (side) light scatter properties. These scatter properties represent the size, shape and granule content of the cells. These properties account for two parameters to be measured as a readout for the viability. Briefly, the DNA of dying or dead cells generally condenses, which alters the 90° scatter; similarly, membrane blebbing can alter the forward scatter. Alterations in the intensity of light scattering, or the cell-refractive index indicate alterations in viability.

Thus, in general, for light scattering assays, a live cell population of a particular cell type is evaluated to determine it's forward and side scattering properties. This sets a standard for scattering that can subsequently be used.

In a preferred embodiment, the viability assay utilizes a viability dye. There are a number of known viability dyes that stain dead or dying cells, but do not stain growing cells. For example, annexin V is a member of a protein family which displays specific binding to phospholipid (phosphotidylserine) in a divalent ion dependent manner. This protein has been widely used for the measurement of apoptosis (programmed cell death) as cell surface exposure of phosphatidylserine is a hallmark early signal of this process. Suitable viability dyes include, but are not limited to, annexin, ethidium homodimer-1, DEAD Red, propidium iodide, SYTOX Green, etc., and others known in the art; see the Molecular Probes Handbook of Fluorescent Probes and Research Chemicals, Haugland, Sixth Edition, hereby incorporated by reference; see Apoptosis Assay on page 285 in particular, and Chapter 16.

Protocols for viability dye staining for cell viability are known, see Molecular Probes catalog, supra. In this embodiment, the viability dye such as annexin is labeled, either directly or indirectly, and combined with a cell population. Annexin is commercially available, i.e., from PharMingen, San Diego, California, or Caltag Laboratories, Millbrae, California. Preferably, the viability dye is provided in a solution wherein the dye is in a concentration of about 100 ng/ml to about 500 ng/ml, more preferably, about 500 ng/ml to about 1 µg/ml, and most preferably, from about 1 µg/ml to about 5 µg/ml. In a preferred embodiment, the viability dye is directly labeled; for example, annexin may be labeled with a fluorochrome such as fluorecein isothiocyanate (FITC), Alexa dyes, TRITC, AMCA, APC, tri-color, Cy-5, and others known in the art or commercially available. In an alternate preferred embodiment, the viability dye is labeled with a first label, such as a hapten such as biotin, and a secondary fluorescent label is used, such as fluorescent streptavidin. Other first and second labeling pairs can be used as will be appreciated by those in the art.

Once added, the viability dye is allowed to incubate with the cells for a period of time, and washed, if necessary. The cells are then sorted as outlined below to remove the non-viable cells.

In a preferred embodiment, exclusion dye staining is used as the viability assay. Exclusion dyes are those which are excluded from living cells, i.e. they are not taken up passively (they do not permeate the cell membrane of a live cell). However, due to the permeability of dead or dying cells, they are taken up by dead cells. Generally, but not always, the exclusion dyes bind to DNA, for example via intercalation. Preferably, the exclusion dye does not fluoresce, or fluoresces poorly, in the absence of DNA; this eliminates the need for a wash step. Alternatively, exclusion dyes that require the use of a secondary label may also be used. Preferred exclusion dyes include, but are not limited to, ethidium bromide; ethidium homodimer-1; propidium iodine; SYTOX green nucleic acid stain; Calcein AM, BCECF AM: fluorescein diacetate; TOTO® and TO-PRO™ (from Molecular Probes: supra, see chapter 16) and others known in the art.

Protocols for exclusion dye staining for cell viability are known, see the Molecular Probes catalog, supra. In general, the exclusion dye is added to the cells at a concentration of from about 100 ng/ml to about 500 ng/ml, more preferably, about 500 ng/ml to about 1 µg/ml, and most preferably, from about 0.1 µg/ml to about 5 µg/ml, with about 0.5 µg/ml being particularly preferred. The cells and the exclusion dye are incubated for some period of time, washed, if necessary, and then the cells sorted as outlined below, to remove non-viable cells from the population.

In addition, there are other cell viability assays which may be run, including for example enzymatic assays, which can measure extracellular enzymatic activity of either live cells (i.e. secreted proteases, etc.), or dead cells (i.e. the presence of intracellular enzymes in the media; for example, intracellular proteases, mitochondrial enzymes, etc.). See the Molecular Probes Handbook of Fluorescent Probes and Research Chemicals, Haugland, Sixth Edition, hereby incorporated by reference; see chapter 16 in particular.

In a preferred embodiment, at least one cell viability assay is run, with at least two different cell viability assays being preferred, when the fluors are compatible. When only 1 viability assay is run, a preferred embodiment utilizes light scattering assays (both forward and side scattering). When two viability assays are run, preferred embodiments utilize light scattering and dye exclusion, with light scattering and viability dye staining also possible, and all three being done in some cases as well. Viability assays thus allow the separation of viable cells from non-viable or dying cells.

In addition to a cell viability assay, a preferred embodiment utilizes a cell proliferation assay. By "proliferation assay" herein is meant an assay that allows the determination that a cell population is either proliferating, i.e. replicating, or not replicating.

In a preferred embodiment, the proliferation assay is a dye inclusion assay. A dye inclusion assay relies on dilution effects to distinguish between cell phases. Briefly, a dye (generally a fluorescent dye as outlined below) is introduced to cells and taken up by the cells. Once taken up, the dye is trapped in the cell, and does not diffuse out. As the cell population divides, the dye is proportionally diluted. That is, after the introduction of the inclusion dye, the cells are allowed to incubate for some period of time; cells that lose fluorescence over time are dividing, and the cells that remain fluorescent are arrested in a non-growth phase.

Generally, the introduction of the inclusion dye may be done in one of two ways. Either the dye cannot passively enter the cells (e.g. it is charged), and the cells must be treated to take up the dye: for example through the use of a electric pulse. Alternatively, the dye can passively enter the cells, but once taken up, it is modified such that it cannot diffuse out of the cells. For example, enzymatic modification of the inclusion dye may render it charged, and thus unable to diffuse out of the cells. For example, the Molecular Probes CellTracker™ dyes are fluorescent chloromethyl derivatives that freely diffuse into cells, and then glutathione S-transferase-mediated reaction produces membrane impermeant dyes.

Suitable inclusion dyes include, but are not limited to, the Molecular Probes line of CellTracker™ dyes , including, but not limited to CellTracker™ Blue, CellTracker™ Yellow-Green, CellTracker™ Green, CellTracker™ Orange, PKH26 (Sigma), and others known in the art; see the Molecular Probes Handbook, supra; chapter 15 in particular.

In general, inclusion dyes are provided to the cells at a concentration ranging from about 100 ng/ml to about 5 µg/ml, with from about 500 ng/ml to about 1 µg/ml being preferred. A wash step may or may not be used. In a preferred embodiment, a candidate bioactive agent is combined with the cells as described herein. The cells and the inclusion dye are incubated for some period of time, to allow cell division and thus dye dilution. The length of time will depend on the cell cycle time for the particular cells; in general, at least about 2 cell divisions are preferred, with at least about 3 being particularly preferred and at least about 4 being especially preferred. The cells are then sorted as outlined below, to create populations of cells that are replicating and those that are not. As will be appreciated by those in the art, in some cases, for example when screening for anti-proliferation agents, the bright (i.e. fluorescent) cells are collected; in other embodiments, for example for screening for proliferation agents, the low fluorescence cells are collected. Alterations are determined by measuring the fluorescence at either different time points or in different cell populations, and comparing the determinations to one another or to standards.

In a preferred embodiment, the proliferation assay is an antimetabolite assay. In general, antimetabolite assays find the most use when agents that cause cellular arrest in G1 or G2 resting phase is desired. In an antimetabolite proliferation assay, the use of a toxic antimetabolite that will kill dividing cells will result in survival of only those cells that are not dividing. Suitable antimetabolites include, but are not limited to, standard chemotherapeutic agents such as methotrexate, cisplatin, taxol, hydroxyurea, nucleotide analogs such as AraC, etc. In addition, antimetabolite assays may include the use of genes that cause cell death upon expression.

The concentration at which the antimetabolite is added will depend on the toxicity of the particular antimetabolite, and will be determined as is known in the art. The antimetabolite is added and the cells are generally incubated for some period of time; again, the exact period of time will depend on the characteristics and identity of the antimetabolite as well as the cell cycle time of the particular cell population. Generally, a time sufficient for at least one cell division to occur.

In a preferred embodiment, at least one proliferation assay is run, with more than one being preferred. Thus, a proliferation assay results in a population of proliferating cells and a population of arrested cells.

In a preferred embodiment, either after or simultaneously with one or more of the proliferation assays outlined above, at least one cell phase assay is done. A "cell phase" assay determines at which cell phase the cells are arrested, M, G1, S, or G2.

In a preferred embodiment, the cell phase assay is a DNA binding dye assay. Briefly, a DNA binding dye is introduced to the cells, and taken up passively. Once inside the cell, the DNA binding dye binds to DNA, generally by intercalation, although in some cases, the dyes can be either major or minor groove binding compounds. The amount of dye is thus directly correlated to the amount of DNA in the cell, which varies by cell phase; G2 and M phase cells have twice the DNA content of G1 phase cells, and S phase cells have an intermediate amount, depending on at what point in S phase the cells are. Suitable DNA binding dyes are permeant, and include, but are not limited to, Hoechst 33342 and 33258, acridine orange, 7-AAD, LDS 751, DAPI, and SYTO 16, Molecular Probes Handbook, supra; chapters 8 and 16 in particular.

In general, the DNA binding dyes are added in concentrations ranging from about 1 µg/ml to about 5 µg/ml. The dyes are added to the cells and allowed to incubate for some period of time; the length of time will depend in part on the dye chosen. In one embodiment, measurements are taken immediately after addition of the dye. The cells are then sorted as outlined below, to create populations of cells that contain different amounts of dye, and thus different amounts of DNA; in this way, cells that are replicating are separated from those that are not. As will be appreciated by those in the art, in some cases, for example when screening for anti-proliferation agents, cells with the least fluorescence (and thus a single copy of the genome) can be separated from those that are replicating and thus contain more than a single genome of DNA. Alterations are determined by measuring the fluorescence at either different time points or in different cell populations, and comparing the determinations to one another or to standards.

In a preferred embodiment, the cell phase assay is a cyclin destruction assay. In this embodiment, prior to screening (and generally prior to the introduction of a candidate bioactive agent, as outlined below), a fusion nucleic acid is introduced to the cells. The fusion nucleic acid comprises nucleic acid encoding a cyclin destruction box and a nucleic acid encoding a detectable molecule. "Cyclin destruction boxes" are known in the art and are sequences that cause destruction via the ubiquitination pathway of proteins containing the boxes during particular cell phases. That is, for example, G1 cyclins may be stable during G1 phase but degraded during S phase due to the presence of a G1 cyclin destruction box. Thus, by linking a cyclin destruction box to a detectable molecule, for example green fluorescent protein, the presence or absence of the detectable molecule can serve to identify the cell phase of the cell population. In a preferred embodiment, multiple boxes are used, preferably each with a different fluor, such that detection of the cell phase can occur.

A number of cyclin destruction boxes are known in the art, for example, cyclin A has a destruction box comprising the sequence RTVLGVIGD; the destruction box of cyclin B1 comprises the sequence RTALGDIGN. See Glotzer et al., Nature 349:132-138 (1991). Other destruction boxes are known as well: YMTVSIIDRFMQDSCVPKKMLQLVGVT (rat cyclin B); KFRLLQETMYMTVSIIDRFMQNSCVPKK (mouse cyclin B); RAILIDWLIQVQMKFRLLQETMYMTVS (mouse cyclin B1); DRFLQAQLVCRKKLQVVGITALLLASK (mouse cyclin B2); and MSVLRGKLQLVGTAAMLL (mouse cyclin A2).

The nucleic acid encoding the cyclin destruction box is operably linked to nucleic acid encoding a detectable molecule. The fusion proteins are constructed by methods known in the art. For example, the nucleic acids encoding the destruction box is ligated to a nucleic acid encoding a p- or rGFP protein.

Accordingly, the results of sorting after cell phase assays generally result in at least two populations of cells that are in different cell phases.

In a preferred embodiment, the methods are used to screen candidate bioactive agents for the ability to modulate cell cycle regulation, including the activation or suppression of cell cycle checkpoint pathways and ameliorating checkpoint defects. The candidate bioactive agent can be added to the cell population exogenously or can be introduced into the cells as described further herein.

In a preferred embodiment, the methods are used to screen candidate bioactive agents for the ability to modulate cell cycle regulation, including the activation or suppression of cell cycle checkpoint pathways and ameliorating checkpoint defects. The candidate bioactive agent can be added to the cell population exogenously or can be introduced into the cells as described further herein.

As above, when the candidate agents are nucleic acids or peptides, fusion partners, are defined herein, may be used. The fusion partners, including presentation structures, may be modified, randomized, and/or matured to alter the presentation orientation of the randomized expression product. For example, determinants at the base of the loop may be modified to slightly modify the internal loop peptide tertiary structure, which maintaining the randomized amino acid sequence.

In a preferred embodiment, combinations of fusion partners are used. Thus, for example, any number of combinations of presentation structures, targeting sequences, rescue sequences, and stability sequences may be used, with or without linker sequences.

Thus, candidate agents can include these components, and may then be used to generate a library of fragments, each containing a different random nucleotide sequence that may encode a different peptide. The ligation products are then transformed into bacteria, such as *E. coli,* and DNA is prepared from the resulting library, as is generally outlined in Kitamura, PNAS USA 92:9146-9150 (1995), hereby expressly incorporated by reference.

Delivery of the library DNA into a retroviral packaging system results in conversion to infectious virus. Suitable retroviral packaging system cell lines include, but are not limited to, the Bing and BOSC23 cell lines described in WO 94/19478; Soneoka et al., Nucleic Acid Res. 23(4):628 (1995); Finer et al., Blood 83:43 (1994); Pheonix packaging lines such as PhiNX-eco and PhiNX-ampho, described below; 292T + gag-pol and retrovirus envelope; PA317; and cell lines outlined in Markowitz et al., Virology 167:400 (1988), Markowitz et al., J. Virol. 62:1120 (1988), Li et al., PNAS USA 93:11658 (1996), Kinsella et al., Human Gene Therapy 7:1405 (1996), all of which are incorporated by reference. Preferred systems include PhiNX-eco and PhiNX-ampho or similar cell lines, disclosed in PCT US97/01019.

When the cells are not replicating, other viral vectors may be used, including adenoviral vectors, feline immunoviral (FIV) vectors, etc. Thus, in a preferred embodiment, adenoviral vectors comprising a rGFP gene are provided. Similarly, FIV vectors comprising an rGFP gene are provided.

In a preferred embodiment, when the candidate agent is introduced to the cells using a viral vector, the candidate peptide agent is linked to an rGFP gene, and the methods of the invention include at least one expression assay. An expression assay is an assay that allows the determination of whether a candidate bioactive agent has been expressed, i.e. whether a candidate peptide agent is present in the cell. Thus, by linking the expression of a candidate agent to the expression of rGFP protein, the presence or absence of the candidate peptide agent may be determined. Accordingly, in this embodiment, the candidate agent is operably linked to a detectable molecule. Generally, this is done by creating a fusion nucleic acid. The fusion nucleic acid comprises a first nucleic acid encoding the candidate bioactive agent (which can include fusion partners, as outlined above), and a second nucleic acid encoding a detectable molecule. The terms "first" and "second" are not meant to confer an orientation of the sequences with respect to 5'-3' orientation of the fusion nucleic acid. For example, assuming a 5'-3' orientation of the fusion sequence, the first nucleic acid may be located either 5' to the second nucleic acid, or 3' to the second nucleic acid. A Preferred detectable molecule in this embodiment includes, but is not limited to, rGFP.

In general, the candidate agents are added to the cells (either extracellularly or intracellularly, as outlined above) under reaction conditions that favor agent-target interactions. Generally, this will be physiological conditions. Incubations may be performed at any temperature which facilitates optimal activity, typically between 4 and 40°C. Incubation periods are selected for optimum activity, but may also be optimized to facilitate rapid high through put screening. Typically between 0.1 and 1 hour will be sufficient. Excess reagent is generally removed or washed away.

A variety of other reagents may be included in the assays. These include reagents like salts, neutral proteins, e.g. albumin, detergents, etc which may be used to facilitate optimal protein-protein binding and/or reduce non-specific or background interactions. Also reagents that otherwise improve the efficiency of the assay, such as protease inhibitors, nuclease inhibitors, anti-microbial agents, etc., may be used. The mixture of components may be added in any order that provides for detection. Washing or rinsing the cells will be done as will be appreciated by those in the art at different times, and may include the use of filtration and centrifugation. When second labeling moieties (also referred to herein as "secondary labels") are used, they are preferably added after excess non-bound target molecules are removed, in order to reduce non-specific binding: however, under some circumstances, all the components may be added simultaneously.

In a preferred embodiment, the cells are sorted using fluorescent-activated cell sorting (FACS). In the invention herein, cell cycle regulation is evaluated by multiple parameters which results in reduced background and greater specificity. In contrast, FACS has been used in the past to evaluate two different or unrelated characteristics at the same time which identifies cells having those two characteristics, but does not reduce the background for the combined characteristics.

Thus, the cells are sorted or enriched in a FACS on the basis of one or more of the assays, including a cell viability assay, a proliferation assay, a cell phase assay, and (when candidate agents are expressed with detectable moieties) an expression assay. The results from one or more of these assays are compared to cells that were not exposed to the candidate bioactive agent, or to the same cells prior to introduction of the candidate agent. Alterations in these results can indicate that said agent modulates cell cycle regulation.

A strength of the present invention is that a library of candidate agents may be tested in a library of cells, because the present methods allow single cell sorting, with extremely high specificity, such that very rare events may be detected. The use of multiple laser paths allows sort accuracy of 1 in 10⁶ with better than 70% accuracy.

In addition, the present invention can, in addition to the identification of multiple cell cycle regulation properties, be combined with the identification of other cellular characteristics. For example, parameters of general cellular health can be determined and selected for by using i.e., dye Indo-1 indicating a calcium response. Other cellular parameters which are routinely identified by the skilled artisan include but are not limited to: cell size, cell shape, redox state, DNA content, nucleic acid sequence, chromatin structure, RNA content, total protein, antigens, lipids, surface proteins, intracellular receptors, oxidative metabolism, DNA synthesis and degradation and intracellular pH.

In a preferred embodiment, each of the measurements is determined simultaneously from an individual cell as it passes through the beam paths of multiple lasers. Alternatively, the measurements are done sequentially. By using more than one parameter to detect cell cycle regulation or alterations in cell cycle regulation, background is reduced and specificity is increased. The cells meeting the parameters of the desired properties can be physically sorted from cells not meeting the desired parameters or they can be identified by their percentage in the cell population.

In general, K_{D} s of ≤ 1 µM are preferred, to allow for retention of binding in the presence of the shear forces present in FACS sorting. In a preferred embodiment, the cells are sorted at very high speeds, for example greater than about 5,000 sorting events per sec, with greater than about 10,000 sorting events per sec being preferred, and greater than about 25,000 sorting events per second being particularly preferred, with speeds of greater than about 50,000 to 100,000 being especially preferred.

Cells processed for stimulation and staining are generally taken up in buffer and filtered prior to cytometry. Cells can be analyzed using a FACSCAN (Becton Dickinson Inc., laser line 488nm) or a Mo-Flo (Cytomation, Inc., laser lines 350nM broadband (UV), 488nm, and 647nm) Cytometer. Cells are sorted, if desired, using the Mo-Flo.

Wherein the cells are analyzed by microscopy, cells post stimulation or staining are generally mounted onto glass slides and coverslipped; these are directly visualized by brightfield and fluorescence microscopy on an inverted microscope (i.e., TE300, Nikon) using standard filter sets. Images can also be obtained using an inverted confocal scanning microscope (Zeiss, Inc" Bio-Rad, Inc.) using standard filter sets.

The sorting results in a population of cells having the desired properties. In a preferred embodiment, the parameters are set to identify at least one candidate bioactive agent that modulates cell cycle regulation.

In a preferred embodiment, the bioactive agent is characterized. This will proceed as will be appreciated by those in the art, and generally includes an analysis of the structure, identity, binding affinity and function of the agent. Generally, once identified, the bioactive agent is resynthesized and combined with the target cell to verify the cell cycle regulation modulation under various conditions and in the presence or absence of other various agents. The bioactive can be prepared in a therapeutically effective amount to modulate cell cycle regulation and combined with a suitable pharmaceutical carrier.

In a preferred embodiment, the cell populations can be subjected to various experimental conditions, with and without the candidate agents. Changes in conditions include but are not limited to changes in pH, temperature, buffer or salt concentration, etc. In a preferred embodiment, the pH is changed, generally by increasing or decreasing the pH, usually by from about 0.5 to about 3 pH units. Alternatively, the temperature is altered, with increases or decreases of from about 5°C to about 30 °C being preferred. Similarly, the salt concentration may be modified, with increases or decreases of from about 0.1 M to about 2 M being preferred.

It is understood by the skilled artisan that the steps of the assays provided herein can vary in order. It is also understood, however, that while various options (of compounds, properties selected or order of steps) are provided herein, the options are also each provided individually, and can each be individually segregated from the other options provided herein. Moreover, steps which are obvious and known in the art that will increase the sensitivity of the assay are intended to be within the scope of this invention. For example, there may be additionally washing steps, or segregation, isolation steps. Moreover, it is understood that in some cases detection is in the cells, but can also take place in the media, or vice versa.

In a preferred embodiment, the cellular phenotype is exocytosis, and the methods and compositions of the invention are directed to the detection of alterations in exocytosis, again using a FACS machine. There are a number of parameters that may be evaluated or assayed to allow the detection of alterations in exocytotic pathways, including, but not limited to, light scattering, fluorescent dye uptake, fluorescent dye release, granule exposure, surface granule enzyme activity, and the quantity of granule specific proteins. By assaying or measuring one or more of these parameters, it is possible to detect not only alterations in exocytosis, but alterations of different steps of the exocytotic pathway. In addition, multiparameter analysis also reduces the background, or "false positives", that are detected. In this manner, rapid, accurate screening of candidate agents may be performed to identify agents that modulate exocytosis.

In a preferred embodiment, the invention provides methods for screening for alterations in exocytosis of a population of cells. By "alteration" or "modulation" in the context of exocytosis is meant a decrease or an increase in the amount of exocytosis in one cell compared to another cell or in the same cell under different conditions. The measurements can be determined wherein all of the conditions are the same for each measurement, or under various conditions, with or without bioactive agents, or at different stages of the exocytic process. For example, a measurement of exocytosis can be determined in a cell population wherein a candidate bioactive agent is present and wherein the candidate bioactive agent is absent. In another example, the measurements of exocytosis are determined wherein the condition or environment of the populations of cells differ from one another. For example, the cells may be evaluated in the presence or absence of physiological signals, such as exocytic inducers (i.e, Ca⁺⁺, ionomycin, etc.), hormones, antibodies, peptides, antigens, cytokines, growth factors, action potentials, or other cells (i.e. cell-cell contacts). In another example, the measurements of exocytosis are determined at different stages of the exocytic process. In yet another example, the measurements of exocytosis are taken wherein the conditions are the same, and the alterations are between one cell or cell population and another cell or cell population.

By a "population of cells" herein is meant a sample of cells as defined above. In this embodiment, the cells are preferably (but not required) to be rapidly growing, retrovirally infectable, and compatible with dyes and antibodies. Preferred cell types for use in this embodiment, include, but are not limited to, mast cells, neurons, adrenal chromaffin cells, basophils, endocrine cells including pancreatic β-cells, pancreatic acinar cells including exocrine cells, neutrophils, monocytes, lymphocytes, mammary cells, sperm, egg cells and PMN leukocytes, endothelial cells, adipocytes, and muscle cells.

The exocytotic methods comprise sorting the cells in a FACS machine by assaying for alterations in at least three of the properties selected from the group consisting of light scattering, fluorescent dye uptake, fluorescent dye release, granule exposure, surface granule enzyme activity, and the quantity of granule specific proteins. In a preferred embodiment, each of the measurements is determined simultaneously from an individual cell as it passes through the beam paths of multiple lasers. Alternatively, the measurements are done sequentially. By using more than one parameter to detect exocytosis or alterations in exocytosis, background is reduced and specificity is increased. The cells meeting the parameters of the desired properties can be physically sorted from cells not meeting the desired parameters or they can be identified by their percentage in the cell population.

In a preferred embodiment, changes in light scattering are assayed to determine alterations in exocytosis in a population of cells. When viewed in the FACS, cells have particular characteristics as measured by their forward and 90 degree (side) light scatter properties. These scatter properties represent the size, shape and granule content of the cells. Upon activation of the cells with a pro-exocytic stimulus, both the forward and side scatter properties of the cells changes considerably. These properties account for two parameters to be measured as a readout for the exocytic event. These properties change in proportion to the extent of exocytosis of the cells and depend on the time course of the exocytic events as well. Alterations in the intensity of light scattering, or the cell-refractive index indicate alterations in exocytosis either in the same cell at different times, or compared to the same cell under different conditions or with candidate bioactive agents present or absent, or compared to different cells or cell populations.

In one embodiment provided herein, a cell population is combined with an agent which is known to stimulate exocytosis and the light scattering properties are determined. Cells having light scattering properties indicating the desirable-exocytic activity can be identified and/or sorted. Exocytic activity as used herein includes lack of activity. In a preferred embodiment, candidate bioactive agents are combined with the cell population prior to or with the exocytic stimulus, as is more fully outlined below. In this embodiment, where light scattering properties differ as between a) a cell population combined with a known exocytic stimulus and a candidate bioactive agent, and b) a cell population combined with a known exocytic stimulus wherein the candidate bioactive agent is absent, it can be determined that the candidate bioactive agent modulates exocytosis. It may also be desirable in some cases to include an inhibitor of exocytosis or to exclude the exocytic stimulus to identify bioactive agents which induce exocytosis. Preferably, light scattering properties are measured in combination with at least one, and preferably two other properties which indicate exocytosis activity. General methodologies for light scattering measurements are further described in Perretti, et al., J. Pharmacol. Methods, 23(3):187-194 (1990) and Hide et al., J. Cell Biol., 123(3):585-593 (1993), both incorporated herein by reference. In general, changes of at least about 5% from baseline are preferred, with at least about 25% being more preferred, at least about 50% being particularly preferred, and at least about 75 to 100% being especially preferred. Baseline in this case generally means the light scatter properties of the cells prior to exocytotic stimulation. In each case provided herein, the baseline may also be set for any control parameter. For example, the baseline may be set at the exocytosis measurement of a particular cell, a similar cell under different conditions, or at a particular time point during exocytosis.

In another preferred embodiment, changes in fluorescent dye uptake are evaluated. Preferred fluorescent dyes include styryl dyes, which indicate exocytosis activity in relation to endocytosis, sometimes referred to as coupled endocytosis. The theory behind coupled endocytosis is that cells undergoing exocytosis must also undergo endocytosis in order to maintain cell volume and membrane integrity. Thus, upon exocytic stimulation, endocytosis is also increased, providing an indirect measurement of exocytosis by quantifying the amount of styryl dye uptake.

In an embodiment provided herein, the cells are bathed in a solution of styryl dye and stimulated with a pro-exocytic stimulus and the dye is quantitated. Preferably, after exocytic stimulation, the cells are spun down, aspirated and resuspended in fresh buffer. In a preferred embodiment, a candidate bioactive agent is combined with the cells as described herein. In some cases, the candidate bioactive agent can be combined with the cells with an inhibitor of exocytosis or without the pro-exocytic stimulus. Preferably, a pro-exocytic stimulus is added to the cell population which results in a dramatic increase in the fluorescence signal of the dye. The increased cell associated signal is due to coupled endocytosis of the styryl dye and is proportional to the exocytic response in both time and intensity. Conversely, the signal is not increased wherein exocytosis is inhibited or is not induced. Alterations are determined by measuring the fluorescence at either different time points or in different cell populations, and comparing the determinations to one another or to standards. In general, changes of at least about 50% from baseline are preferred, with changes of at least about 75%-100% being more preferred, changes of at least about 250% being particularly preferred, and changes of at least about 1000-2000% being especially preferred. Baseline in this case means the styryl dye uptake of cells prior to exocytic stimulation.

Preferred styryl dyes include, but are not limited to FM1-43, FM4-64, FM14-68, FM2-10, FM4-84, FM1-84, FM14-27, FM14-29, FM3-25, FM3-14, FM5-55, RH414, FM6-55, FM10-75, FM1-81, FM9-49, FM4-95, FM4-59, FM9-40, and combinations thereof. Preferred dyes such as FM1-43 are only weakly fluorescent in water but very fluorescent when associated with a membrane, such that dye uptake is readily discemable. Suitable dyes are available commercially, i.e., Molecular Probes, Inc., of Eugene, Oregon, "Handbook of Fluorescent Probes and Research Chemicals", 6th Edition, 1996, particularly, Chapter 17, and more particularly, Section 2 of Chapter 17, (including referenced related chapter), hereby incorporated herein by reference. Preferably, the dyes are provided in a solution wherein the dye concentration is about 25 to 1000- 5000 nM, with from about 50 to about 1000 nM being preferred, and from about 50 to 250 being particularly preferred. The use of styryl dyes is further described in Betz, et al., Current Opinion in Neurobiology, 6:365-371 (1996) also incorporated herein by reference. Preferably, fluorescent dye uptake is measured in combination with at least one, and preferably two other indicators of exocytosis activity.

In another preferred embodiment, changes in fluorescent dye release are evaluated. The present invention is in part directed to the discovery that low pH concentration dyes, which are normally used to stain lysozomes, also low pH stain exocytic granules. Generally, these dyes can be taken up by the cells passively and concentrate in granules; however, the cells can be induced to take up the dye, i.e., by coupled endocytosis. In a preferred embodiment, a cell population is bathed in a low pH concentration dye such that the dye is taken up by the cells. The cells are preferably washed. The cells can be exposed to a pro-xocytic stimulus and/or inhibitor. In a preferred embodiment, a candidate bioactive agent is combined with the cell population and preferably, the pro-exocytic stimulus. Fluorescence is evaluated. Changes in fluorescent dye release between cells or at different time points in the same cell indicate alterations in exocytosis. Preferably, the alterations are between cells, and most preferably, between cells having different bioactive agents added thereto. Changes of at least about 5% from baseline are preferred, with at least about 25% being more preferred, at least about 50% being particularly preferred and at least about 100% being especially preferred. Baseline in this case means the amount of dye in the cells prior to stimulation.

In this embodiment, low pH concentration dyes are preferred. Such low pH concentration dyes include but are not limited to acridine orange, LYSOTRACKER™ red, LYSOTRACKER™ green, and LYSOTRACKER™ blue. Such dyes are commercially available, i.e., from Molecular Probes, supra, particularly including Chapter 17, Section 4 of Chapter 17, and referenced "related chapters", i.e., Chapter 23. In preferred embodiments, the dyes are administered in a solution wherein the dye is a concentration of about 50 nM to about 25 µM, with from about 5 µM to about 25 µM being preferred, and from about 1 to 5 µM being particularly preferred. The use of low pH concentration dyes is generally described (in regards to lysozome studies) in Haller, et al., Cell Calcium, 19(2):157-165 (1996), hereby incorporated herein by reference.

In an alternative embodiment wherein changes in fluorescent dye release are evaluated, the fluorescence released into the supernatant is evaluated. In this embodiment, either styryl dyes, which reversibly label endocytosed membranes, or low pH concentration dyes are used. In this embodiment, a cell population is bathed in dye such that the dye is taken up into the cells passively or by induction. The cells are then preferably washed. The cells can be exposed to a pro-exocytic stimulus and/or inhibitor, and optionally, a candidate bioactive agent. The cells which are exposed to a pro-exocytic stimulus will release the dye into the extracellular medium. The fluorescence in the medium can be measured or detected. This process is sometimes referred to as destaining the cells. Optionally, an agent for improving and facilitating the detection of the dye in the medium can be added. For example, micelle-forming detergents such as 3-[(3-cholamidopropyl)dimethylammonio]-1-propanesulfonate (CHAPS) increase the fluorescence and thereby allow detection of small amounts of exocytosis activity. Changes in the release of dye will indicate alterations in exocytosis in the same cell, between cells, and most preferably, between cells having different bioactive agents added thereto. In general, changes of at least about 5% from baseline are preferred, with at least about 25% being more preferred, with at least about 50% being particularly preferred and at least about 100% being especially preferred. Baseline in this case means the release of dye prior to exocytotic stimulus. Preferably, dye release when measured in the media is combined with the evaluation of at least one other exocytosis indicator.

In a preferred embodiment, changes in granule exposure are determined. The granules are exposed to the media during exocytosis, i.e., the granules fuse with the cell membrane and expose/release their contents. Therefore, granule exposure is indicative of exocytic activity, and its absence is indicative that exocytosis has not been induced, or has been inhibited. Preferably, granule exposure is detected by a detectable agent which specifically bind to granules. An example of a detectable agent used herein is annexin V, a member of a protein family which displays specific binding to phospholipid (phosphotidylserine) in a divalent ion dependent manner. This protein has been widely used for the measurement of apoptosis (programmed cell death) as cell surface exposure of phosphatidylserine is a hallmark early signal of this process. Surprisingly, it has been determined herein that annexin V specifically binds to exocytic granules when they are exposed at the cell surface during the secretory process; granules internal to the cell are unlabeled. This property of annexin V is used herein to create a single exocytosis assay based on its exocytosis dependent binding. Upon exocytic stimulation of cells, the cells show an increase in annexin binding and fluorescent signal in proportion in both time and intensity to the exocytic response.

In this embodiment, annexin is labelled, either directly or indirectly, and combined with a cell population. Annexin is commercially available, i.e., from PharMingen, San Diego, California, or Caltag Laboratories, Millbrae, California. Preferably, the annexin is provided in a solution wherein the annexin is in a concentration of about 100 ng/ml to about 500 ng/ml, more preferably, about 500 ng/ml to about 1 µg/ml, and most preferably, from about 1 µg/ml to about 5 µg/ml. In a preferred embodiment, the annexin is directly labelled; for example, annexin may be labelled with a fluorochrome such as fluorecein isothiocyanate (FITC), Alexa dyes, TRITC, AMCA, APC, tri-color, Cy-5, and others known in the art or commercially available. In an alternate preferred embodiment, the annexin is labelled with a first label, such as a hapten such as biotin, and a secondary fluorescent label is used, such as fluorescent streptavidin. Other first and second labelling pairs can be used as will be appreciated by those in the art.

In the preferred embodiment, the cells are subjected to conditions that normally cause exocytosis. Optionally, a candidate bioactive agent is added to the cells. In some cases, it may be desirable to include an inhibitor of exocytosis to determine whether the candidate agent can reverse the inhibition, or to add the candidate bioactive agent without an exocytic stimulus to determine whether the agent induces exocytosis. The cells are preferably washed and fluorescence is detected in the microscope or on the flowcytometer. Alterations in the detection of annexin binding indicates alterations in exocytosis in the same cell, or between different cells, with or with the same conditions and/or agents combined therewith. In general, changes of at least about 25% from baseline are preferred, with at least about 50% being more preferred, at least about 100 being particularly preferred and at least about 500% being especially preferred. Baseline in this case means the amount of annexin binding prior to exocytic stimulation.

In another preferred embodiment, granule exposure is detected by a cationic dye such as berberine or ruthenium red. Such cationic dyes specifically stain secreting granules. Thus, when exocytosis occurs, and secreting granules are exposed at the cell surface, an increase in fluorescence can be detected. In a preferred embodiment, the cationic dye is combined with a cell population in the presence or absence of an exocytic stimulus and/or inhibitor, and optionally, in the presence or absence of a candidate bioactive agent. In a particularly preferred embodiment, the berberine is combined with a cell and an exocytic stimulus and a candidate bioactive agent to determine whether the candidate bioactive agent can modulate the exocytic activity. Preferably, the cells are washed and then fluorescence is determined. In preferred embodiments, cationic dye evaluation is combined with evaluation of at least one other indicator of exocytosis. The dye is combined with the cells as is known in the art. General methodologies describing berberine are described in Berlin and Enerback, Int. Arch. Allergy Appl. Immunol., 73(3):256-262 (1984) hereby incorporated by reference. In general, changes of at least about 5% from baseline are preferred, with at least about 25% being more preferred, at least about 50% being particularly preferred, and at least about 100% being especially preferred. Baseline in this case means the amount of dye binding prior to stimulation.

Similarly, Con A-FITC can be used, as it binds to the carbohydrate on granule proteins, in a manner similar to those outlined herein.

In another preferred embodiment, changes in surface granule enzyme activity is determined. Secretory granules contain enzymes such as proteases and glycosidases which are released as part of the exocytic process. Frequently, these enzymes are inactive within the granule, due to the low pH, but upon exposure to the extracellular media at physiological pH, they become activated. These enzyme activities can be measured using chromogenic or fluorogenic substrates as components of the extracellular media. This allows detection of exocytic cells in varying approaches.

In one embodiment, sometimes called herein the population based enzyme assay, the generation of signal via cleavage of a chromogenic or fluorogenic substrate can be quantified in the media. That is, the amount of detectable reaction product in the media is related to the amount of enzyme present, and thus to the amount of exocytosis. In this embodiment, it is the media, not the cells, that becomes detectable.

In a preferred embodiment, cells are subjected to an exocytic stimulus, and optionally, a candidate bioactive agent. The chromogenic or fluorogenic substrate is added to the media, and changes in the signal are evaluated, as the enzymes cleave the extracellular substrates.

In an alternate preferred embodiment, sometimes called herein "in situ enzymology assay", fluorogenic substrates that precipitate upon cleavage are used. That is, upon exocytosis a considerable amount of enzyme activity remains cell/granule associated and can be visualized using fluorescent substrates which precipitate at the site of activity. For example, substrates for glucuronidase, such as ELF-97 glucuronide, precipitate on exocytosing cells, but not resting cells, and thus the cells can show increased fluorescence. The fluorescence is a direct measurement of exocytosis and is pH dependent reflecting the pH optima of the exocytosed enzyme. This method also provides a method of distinguishing different subtypes of granules based on their enzyme profile.

In a preferred embodiment, the cell population is subjected to an exocytic stimulus and then incubated with a detectable substrate. A candidate bioactive agent is optionally added. The cells are washed and then viewed in the microscope or flowcytometer.

Preferred granule enzymes include but are not limited to chymase, tryptase, arylsulfatase A, beta-hexosaminidase, beta-glucuronidase, and beta-D-galactosidase. Substrates include ELF-97 glucuronide, N-acetyl beta-D glucoronide, ELF-97 coupled to peptides, etc., many of which are commercially available, i.e., from Molecular Probes, supra, particular Chapter 10, more particularly Section 2 of Chapter 10, and referenced "related chapters".

By detectable substrate is meant that the substrate comprises a fluorescent molecule as further described herein, or can be detected with a fluorescent molecule specific for the substrate or cleaved substrate, i.e., a fluorescent antibody. In a preferred embodiment, the substrate comprises a detectable molecule formed of two fluorescent proteins, i.e., blue and green fluorescent protein (BFP and rGFP), and other similar molecules. As is known in the art, constructs of rGFP and BFP that hold these two proteins in close proximity allow fluorescence resonance energy transfer (FRET). That is, the excitation spectra of the rGFP overlaps the emission spectra of the BFP.
Accordingly, exciting the BFP results in rGFP emission. If a protease cleavage site is engineered between the rGFP and BFP to form a "FRET construct", upon exposure of the FRET construct to an active protease which cleaves the construct, the rGFP and BFP molecules separate. Thus, exciting the rGFP results in BFP emission and loss of BFP emission.

Preferably, the protease dependent cleavage site inserted between two fluoroscent proteins of the FRET construct is specific for a granule specific enzyme. Thus, the FRET construct can be used for detecting granule specific proteases specific for the cleavage site of the FRET construct. In this embodiment, the protease substrate that is combined with the cells or media includes the FRET construct. The FRET system allows for detection of the detectable molecule in its cleaved and uncleaved state, and distinguishes between the two. The system is further described in Xu et al., Nucleic Acid Res. 26(8):2034 (1998); and Miyawaki et al., Nature 388(6645):882-887 (1997), both of which are incorporated by reference.

The amount of substrate added to the cells or media will depend in part on the enzyme's specific activity and the substrate itself, but generally is about 250 nM to about 1 mM, from about 1 µM to about 100 µM being preferred, and from about 1 µM to about 10 µM being particularly preferred. In general, changes of at least about 5% from baseline are preferred, with at least about 25% being preferred, at least about 100% being particularly preferred and at least about 1000% being especially preferred. Baseline in this case means the amount of substrate cleavage prior to induction of exocytosis.

In a preferred embodiment, changes in the quantity of granule specific proteins are determined. Secretory granules contain proteins which are specifically targeted to the granule compartment due to specific properties of these proteins. Upon exocytic induction, the granule specific proteins are exposed to the surface and detected.

In a preferred embodiment, detectable granule specific proteins are combined with a population of cells and subjected to conditions known to induce exocytosis. Optionally, a bioactive candidate is combined with the cell population and detectable granule specific protein and the granule specific protein is detected. Granule specific proteins include but are not limited to VAMP and synaptotagmin. Also included within the definition of granule specific proteins are the mediators released during exocytosis, including, but not limited to, serotonin, histamine, heparin, hormones, etc.

The quantification of the granule proteins may be done in several ways. In one embodiment, labelled antibodies, (such as fluoroscent antibodies), to granule specific proteins are used. In another embodiment, the cells are engineered to contain fusion proteins comprising a granule protein and a detectable molecule. In a preferred embodiment, a detectable molecule is added to the cells for detection. For example, either directly or indirectly labelled antibodies can be used. A preferred embodiment uses a first labelled antibody, with fluorescent labels preferred. Another embodiment uses a first and second label, for example, a labelled secondary antibody. Generally, this embodiment may use any agent that will specifically bind to the granule protein or compound that can be either directly or indirectly labelled.

In a preferred embodiment the labels are engineered into the cells. For example, recombinant proteins are introduced to the cell population which are fusion proteins of a granule specific protein and a detectable molecule. This is generally done by transforming the cells with a fusion nucleic acid encoding a fusion protein comprising a granule specific protein and a detectable molecule. This is generally done as is known in the art, and will depend on the cell type. Generally, for mammalian cells, retroviral vectors and methods are preferred.

The fusion proteins are constructed by methods known in the art. For example, the nucleic acids encoding the granule specific protein is ligated with a nucleic acid encoding a detectable molecule. By detectable molecule herein is meant a molecule that allows a cell or compound comprising the detectable molecule to be distinguished from one that does not contain it, i.e., an epitope, sometimes called an antigen TAG, or a fluorescent molecule. Preferred fluorescent molecules include but are not limited to p- or rGFP, BFP, YFP, enzymes including luciferase and β-galactosidase. These constructs can be made in such a way so that upon exocytosis an epitope, internal to the granule, is exposed at the cell surface and can then be detected. The epitope is preferably any detectable peptide which is not generally found on the cytoplasmic membrane, although in some instances, if the epitope is one normally found on the cells, increases may be detected, although this is generally not preferred.

In a preferred embodiment, the cell population containing the fusion protein or detectable granule specific protein is subjected to exocytic conditions. Optionally, a candidate bioactive agent and/or exocytic inhibitor is included. Preferably, the cells are washed. Fluorescence is detected on the cells. In general, changes of at least about 5% from baseline are preferred, with at least about 25% being more preferred, at least about 50% being particularly preferred and at least about 100% being especially preferred. Generally, baseline in this case means amount of fluorescence prior to exocytic stimulus.

In the invention herein, the same characteristic of exocytosis is evaluated by multiple parameters which results in reduced background and greater specificity. In contrast, FACS has been used in the past to evaluate two different or unrelated characteristics at the same time which identifies cells having those two characteristics, but does not reduce the background for the combined characteristics. The present invention can, however, in addition to the identification of multiple exocytosis properties, be combined with the identification of other cellular parameters, as outlined above.

In a preferred embodiment, the cells are subjected to conditions that normally cause exocytosis. Pro-exocytic agents include ionomycin, Ca⁺⁺ , ionophores (lonomycin, AZ3187), compound 48/80, substance P, complement C3a/C5a. trypsin, tryptase, insulin, interleukin-3, specific IgE, allergen, anti-IgE, or anti-IgG receptor antibodies. These are provided at concentrations depending on the compound as is known in the art, ranging from 1 picomolar to 10 µM, generally. In some cases, it may be desirable to combine the cells with agents which inhibit exocytosis. Exocytosis inhibitors include but are not limited to Wortmannin, and Genestein, and others known in the art.

In a preferred embodiment, the methods are used to screen candidate bioactive agents for the ability to modulate exocytosis. The candidate bioactive agents may be combined with the cell population before, during or after exocytosis is stimulated, preferably before. In some instances, it may be desirable to determine the effect of the candidate bioactive agent, also referred to as "candidate agents" herein, on the cell wherein exocytosis is not induced or wherein exocytosis is inhibited. The candidate bioactive agent can be added to the cell population exogenously or can be introduced into the cells as described further herein.

In a preferred embodiment, as above for cell cycle assays, a library of different candidate bioactive agents are used.

As above, the candidate bioactive agents are combined or added to a cell or population of cells; again, as outlined above, preferred embodiments utilize nucleic acid candidate agents and fusion partners; and preferably retroviral constructs.

Wherein the candidate agents are nucleic acids, methods known in the art such as calcium phosphate, electroporation, and injection may be used to introduce these to the cells. The exocytic stimulus is generally combined with the cells under physiological conditions. Incubations may be performed at any temperature which facilitates optimal activity, typically between 4 and 40°C. Incubation periods are selected for optimum activity, but may also be optimized to facilitate rapid high through put screening.

As above, a variety of other reagents may be included in the assays, and the cells are sorted as above. The sorting results in a population of cells having the desired exocytic properties. In a preferred embodiment, the parameters are set to identify at least one candidate bioactive agent that modulates exocytosis.

In a preferred embodiment, the bioactive agent is characterized. This will proceed as will be appreciated by those in the art, and generally includes an analysis of the structure, identity, binding affinity and function of the agent. Generally, once identified, the bioactive agent is resynthesized and combined with the target cell to verify the exocytosis modulation under various conditions and in the presence or absence of other various agents. The bioactive can be prepared in a therapeutically effective amount to modulate exocytosis and combined with a suitable pharmaceutical carrier.

In a preferred embodiment, the cell populations can be subjected to various experimental conditions, with and without the candidate agents, and with and without exocytic stimulation or inhibition. Changes in conditions include but are not limited to changes in pH, temperature, buffer or salt concentration, etc. In a preferred embodiment, the pH is changed, generally by increasing or decreasing the pH, usually by from about 0.5 to about 3 pH units. Alternatively, the temperature is altered, with increases or decreases of from about 5°C to about 30 °C being preferred. Similarly, the salt concentration may be modified, with increases or decreases of from about 0.1 M to about 2 M being preferred.

In a preferred embodiment, the cellular phenotype to be modulated is small molecule (or other candidate agent) toxicity. These are generally as outlined above for cell viability assays. Small molecule dose responses can also be compared by comparing the cells with the greatest functional response, and then backgating to see if there is more or less toxicity associated with those cells.

In a preferred embodiment, the cellular phenotype involves the expression or activity of cell surface receptors; up to sixteen cell surface markers may be followed simultaneously, with up to eight being preferred. The presence or absence of any particular cell surface marker can be detected by directly and indirectly conjugated antibodies against any cell surface protein whose cell surface expression reflects an important functional parameter associated with the cells being studied. The effect of candidate agents such as small molecules can then be tested against individual or multiple markers.

In a preferred embodiment, the cellular phenotype involves the expression or activity of enzymes such as fluorescent based reporter systems that can report a biological event that occurs simultaneously with the primary measurement or is a result of the primary measurement. This reporter system can be a readout of upstream signal transduction pathways that are active in the cytoplasm, or of nuclear transcriptional or translational events, as well as export events from the nucleus or the cell.

In a preferred embodiment, the cellular phenotype involves protein-protein interactions (or interactions between other binding ligands), such as dimerization, that can be either disrupted or instigated by a candidate agent. These events may be measured by the appearance or disappearance of FRET between two labeled binding ligands.

All references cited herein are incorporated by reference.

### EXAMPLES

### Vector Construction

Retroviral constructs were based on a pCGFP vector that carries a composite CMV promoter fused to the transcriptional start site to the MMLV R-U5 region of the LTR, and extended packaging sequence, deletion of the MMLV gag start ATG, and a multiple cloning region encoding human codon-optimized EGFP (Clontech, Palo Alto, CA) and a Kozak consensus start, described in Kozak *Cell* 44:283-292. The vector used to express flag tagged EGFP, pEf, is identical to pCGFP but has additional restriction sites in the open reading frame of EGFP (resulting in 8 non-human optimized codons) and a Flag tag fused to the C-terminus of EGFP with the linker EEAAKA.

pR and pP are retroviral expression vectors containing human codon-optimized *Renilla muelleri* and *Ptilosarcus gurneyi* GFPs (containing 9 and 11 non-optimized codons, respectively, to introduce restriction sites). Each has a Kozak consensus start and backbone vector sequence identical to that of pCGFP and pEf. These vectors were made by annealing and ligating 20 synthetic oligonucleotides (10 forward, 10 reverse for each GFP gene) creating a dsDNA fragment for each sequence shown in Table 1. These fragments were PCR amplified with respective primers:
R forward, 5' -
GATCATAGAATTCGCCACCATGGGCAGCAAGCAGATCCTGAAGAACACCTGCCTG;
P forward, 5'-
GATCATAGAATTCGCCACCATGGGCAACCGCAACGTGCTGAAGAACACCGGCCTG;
R and P reverse, 5'-
ATGATCGCGGCCGCTACACCCACTCGTGCAGGGATCCCAGGGGCTTGCCGATG;
and cloned into the EcoRl/Notl restriction sites of pEf (replacing the Ef coding region). C-terminal Flag tags were added to these GFPs through BamHl/Notl sites using annealed primers with sticky overhangs:
Forward, 5' -
GATCCCTGCACGAGTGGGTGGAGGAGGCCGCCAAGGCCGACTACAAGGACGACGACGACAAG
TAGGCCCGTGAGGCCCTAAGC;
Reverse, 5' -
GGCCGCTTAGGGCCTCACGGGCCTACTTGTCGTCGTCCTTGTAGTCGGCCTTGGCGGCCTCCT
CCACCCACTCGTGCAGG;
creating Rf and Pf. pRcDNA was made by removing the *R. muelleri* cDNA gene from pET-34 Native *Renilla muelleri* GFP (Prolume Ltd., Pittsburg, PA) by PCR amplification with primers:
Forward, 5' -
GATCATGAATTCGCCATGAGTAAACAAATATTGAAGAACACT;
Reverse, 5' -
TAGATCGCGGCCGCTTAAACCCATTCGTGTAAGGATCCTAGTGG;
and cloning into the EcoRI/NotI sites of pEf. Vectors containing codon optimized *R. muelleri* GFP with a linker-HA tag-linker sequence inserted into each position A-F were created by the PCR sew technique of two fragments using primers shown above (R forward and R reverse). The two fragments for A-F were made by PCR amplification of the 5' section of R with respective primers:
R forward, shown above;
A reverse, 5'-
CTGGCGTAGTCGGGCACGTCGTAGGGGTAGCCACCGCCCTGGCCCTCGTAGCGCAGGGTGCG
CTCGTAC;
B reverse, 5' -
CTGGCGTAGTCGGGCACGTCGTAGGGGTAGCCACCGCCCTGGCCCTCGATCAGGTTGATGTCG
CTGCGG;
C reverse, 5' -
CTGGCGTAGTCGGGCACGTCGTAGGGGTAGCCACCGCCCTGGCCGTTCATGTACATGGCCTCG
AAGCTG;
D reverse, 5' -
CTGGCGTAGTCGGGCACGTCGTAGGGGTAGCCACCGCCCTGGCCGTTAAGCTTGTACACAGGA
TCACC;
E reverse, 5' -
CTGGCGTAGTCGGGACGTCGTAGGGGTAGCCACCGAAATGGAAGAAATTGCTCTTCATCAGGG
TCTTC;
F reverse, 5' -
CTGGCGTAGTCGGGCACGTCGTAGGGGTAGCCACCGCCCTGGCCGCCGCCGTCCTCCACGTA
GGTCTTC;
and the 3' section of R with respective primers:
A forward, 5'-
CCTACGACGTGCCCGACTACGCCAGCCTGGGCCAGCAGGTGGAGGCGACGGCGGCCTGGTGG
AGATCCGCA;
B forward, 5' -
CCTACGACGTGCCCGACTAGCCAGCCTGGGCCAAGCAGGTGGAGGCGACAAGTTCGTGTACCG
CGTGGAGT;
C forward, 5' -
CCTACGACGTGCCCGACTACGCCAGCCTGGGCCAAGCAGGTGGAGGCAACGGCGTGCTGGTG
GGCGAGGTGA;
D forward, 5' -
CCTACGACGTGCCCGACTACGCCAGCCTGGGCCAAGCAGGTGGAGGCAGCGGCAAGTACTACA
GCTGCCACA;
E forward, 5'-
CCTACGACGTGCCCGACTACGCCAGCCTGGGCCAAGCAGGTGGAGGCGTGGTGAAGGAGTTC
CCCAGCTACC;
F forward, 5'-
CCTACGACGTGCCCGACTACGCCAGCCTGGGCCAAGCAGGTGGAGGCTTCGTGGAGCAGCAC
GAGACCGCCA. The PCR sewed fragments were put into the EcoRI/NotI sites of pEf.

The bacterial expression vector for purification of *Ptilosarcus* GFP was created by PCR amplification of pP with primers:
forward, 5' -
AGATCATAGATCTATGGGCAACCGCAACGTGCTGAAGAACACCGGCCTG;
P reverse, shown above.

Digestion of the fragment withBgIII/NotI and ligation into the BamHI/NotI restriction sites of pGEX6P-1 (Pharmacia Biotech, Piscataway, New Jersey). The vector containing *R. muelleri* GFP with C10G and C35E mutations (observed to aid in the folding of the protein in bacteria) was created by PCR sewing together a fragment created by annealing and extending primers:
forward, 5' -
AGATCATAGATCTGAATTCATGGGCAGCAAGCAGATCCTGAAGAACACCGGCCTGCAGGAGGTG
ATGAGCTACAAGGTGACCTGGAGG;
reverse, 5' -
GCCAACAGGATGTTGCCCTTGCCCTCGCCCTCCATGGTGAACACGTGGTTGTTAACGATGCCCT
CCAGGTTCACCTTGTAGCTCATCAC;
R reverse, shown above.
The sewed product was digested BgIII/NotI and ligated into the BamHI/NotI sites of pGEX6P-1.

### Cells and Retrovirus Transduction

Phoenix E retroviral packaging cells, described in Swift et al., Current Protocols in Immunology (1999) 10.17C:1-17, were carried in 10% fetal bovine serum with 1% penicillin-streptomycin (JHR Bioscience, Williamsburg, VA) and Dulbecco's modified Eagle media (Mediatech Cellgro, Hemdon, VA). Jurkat cells stably expressing the ecotropic receptor (Jurkat E) were carried in 10% fetal calf serum with 1% penicillin-streptomycin in RPMI 1630 media (JRH Bioscience, Williamsburg, VA). Calcium phosphate transfection of Phoenix E cells and infection of Jurket E cells and infection of Jurket E cells was carried out as described in Swift *et al.*

### Gel Filtration

Gel filtration was carried out on a 1 x 30 cm Pharmacia Superdex 75 column, equilibrated in phosphate buffered saline and eluted at 0.3 ml/min. at 22°C. The column was on a Hewlett-Packard 1100 HPLC system equipped with a standard fluorescence detector with an 8µl flow cell. GFP peaks were detected by absorption at 489nm or by fluorescence emission at 512nm. Fluorescence excitation spectra were recorded with a fixed emission wavelength at 549nm, and emission spectra were recorded at a fixed excitation wavelength of 450nm.

### FACS and Microscopy

Flow-cytometry analysis and cell sorting of GFP expressing cells were performed on a FACScan (Beckton-Dickson, San Jose, CA) or MoFlo (Cytomation, Fort Collins, CO) instrument, and data analyzed using FloJo software (Treestar Software, San Carlos, CA). Live cells were gated on by scatter and propium iodide staining during data analysis. GFP fluorescence intensity measurements (Geometric mean) were of GFP positive cells only. Cells expressing GFP were visualized using Nikon Ellipse TE300 fluorescence microscope.

### Western Analysis

For preparation of whole-cell lysates, identical numbers of cells were collected, washed in PBS and prepared in lysis buffer (50mM Hepes pH 7.4, 150mM NaCl, 5mM EDTA, 5mM EGTA, 1% TritonX-100) with added Complete EDTA-free protease inhibitor cocktail (Boehringer Mannheim, Chicago, II). Lysate cleared by centrifugation was resolved on 4-12% NuPage SDS polyacrylamide gels (Novex, San Diego, CA) as per the manufacturer's recommendations. Samples transferred to PVDF membranes were blotted using 10% Milk, 0.1% Tween20 in 1X PBS blocking buffer with rabbit polyclonal flag-probe (Santa Cruz Biotechnology, Santa Cruz, CA) at a 1:2000 dilution and goat anti-rabbit IgG-horse radish peroxidase conjugate (Sigma, St. Louis, MO) secondary at a 1:5000 dilution. Membranes were detected using ECL plus enhanced chemiluminescence kit (Amersham Pharmacia, Piscataway, NJ) and Hyperfilm ECL film (Amersham Life Sciences, Buckinghamshire, UK). Exposed film was scanned with a Hewlett Packard (Palo Alto, CA) ScanJet 4C scanner and band intensities were integrated using the program NIH Image (see http://rsb.info.nih.govlnih-imacte/about.html).

### GFP Purification from E. coli

All components used for purification of the GFP gene products were from Pharmacia Biotec (Piscataway, NJ) except as noted. The human codon-optimized gene for each protein was expressed in BL21 TIL codon plus (DE3) *E. coli* (Stratagene, San Diego, CA) as a fusion protein with glutathione S-transferase from pGEX6p-1 derived vectors. Each protein was purified using Glutathione Sepharose 4B beads as per the manufacturer's directions, and the mature GFP was removed from the protein with Precision Protease. The purified proteins ran as single bands by SDS-PAGE and appeared as single peaks of the expected molecular mass by MALDI-TOF mass spectometry on a Bruker Reflex III instrument (Bruker Daltonics, Billerica, MA). Due to the cloning strategy, purified R. *muelleri* GFP has the amino acids PLGSEF- and *Ptilosarcus* GFP the residues GPLGS- fused to their N-termini. Purified recombinant EGFP was from Clontech (Palo Alto, CA).

### CD Studies

CD spectra were recorded as described in Gururaja *et al., Chem. Biol.* (2000) in press. Spectra were recorded between 200 and 250nm at 0.2nm intervals with a time constant of 1s. Data was collected from five separate scans and averaged. The protein concentrations were in the range of 5 to 10µM, as determined by the Lowery method, described in Lowry *et al., J*. *Biol. Chem.* (1951) 193:265-275. Protein solutions were made in 10mM phosphate buffer containing 100mM KF at pH 7.5, and were diluted in the same buffer to yield appropriate the final concentration. The thermal denaturation was measured at 218nm over a range of 4-98°C with a temperature step of 2°C, a 2 minute equilibration time, and a 60s signal averaging time. The apparent Tₘ was also determined by fitting the data to a logistic sigmoid equation using the Levenberg-Marquardt algorithm in Ultrafit (Biosoft, Cambridge, UK). In addition, the apparent Tₘ was determined as the maximum of the first derivative of the CD signal with respect to temperature. Both methods of Tₘ calculation agreed well. CD spectra were deconvoluted with the program CDNN (CD neural network) downloaded from http://bioinformatik.biochemtech.uni-halle.de/cdnnlindex.html.

## Claims

1. A retroviral vector comprising a nucleic acid encoding a Renilla GFP.

2. A retroviral vector of claim 1, further comprising an IRES site.

3. A cell comprising a retroviral vector according to claim 1 or 2.

4. A library of retroviral vectors wherein each vector comprises a nucleic acid encoding a Renilla GFP fused to a protein of interest.

5. A library of cells comprising a library of retroviral vectors according to claim 4.

6. A library of cells according to claim 5 wherein said cells are mammalian.

7. A library of retroviral vectors of claims 4 to 6, wherein said protein of interest is a random peptide.

## Patentansprüche

1. Retroviraler Vektor, der Nucleinsäure umfasst, die für ein Renilla-GFP kodiert.

2. Retroviraler Vektor nach Anspruch 1, der weiters eine IRES-Stelle umfasst.

3. Zelle, die einen retroviralen Vektor nach Anspruch 1 oder 2 umfasst.

4. Bibliothek retroviraler Vektoren, worin jeder Vektor Nucleinsäure umfasst, die für ein Renilla-GFP kodiert, das an ein Protein von Interesse fusioniert ist.

5. Bibliothek von Zellen, die eine Bibliothek retroviraler Vektoren nach Anspruch 4 umfassen.

6. Bibliothek von Zellen nach Anspruch 5, worin die Zellen Säugetierzellen sind.

7. Bibliothek retroviraler Vektoren nach einem der Ansprüche 4 bis 6, worin das Protein von Interesse ein Zufallspeptid ist.

## Revendications

1. Vecteur rétroviral comprenant un acide nucléique codant pour GFP de Renilla.

2. Vecteur rétroviral de la revendication 1, comprenant de plus un site IRES.

3. Cellule comprenant un vecteur rétroviral selon la revendication 1 ou 2.

4. Bibliothèque de virus rétroviraux où chaque vecteur comprend un acide nucléique codant pour GFP de Renilla fusionné à une protéine d'intérêt.

5. Bibliothèque de cellules comprenant une bibliothèque de vecteurs rétroviraux selon la revendication 4.

6. Bibliothèque de cellules selon la revendication 5 où lesdites cellules sont mammaliennes.

7. Bibliothèque de vecteurs rétroviraux des revendications 4 à 6, où ladite protéine d'intérêt est/un peptide statistique.
